# EUROPÄISCHE PATENTANMELDUNG

(11) **EP 2 778 220 A1**
(43) Veröffentlichungstag der Anmeldung: **17.09.2014**
(21) Anmeldenummer: 13158634.9
(22) Anmeldetag: 11.03.2013
(51) Int. Cl.: C12N 1/21, C12N 9/42, C12N 15/52, C12N 15/63, C12P 19/14

(54) **Ganzzell-Biokatalysatoren zum Abbau von cellulosischer Biomasse**

(71) Anmelder: ZYRUS Beteiligungsgesellschaft mbH & Co. Patente I KG, 12529 Schönefeld / Waltersdorf (DE)
(72) Erfinder: Jürgens, Dr. Michael, 30629 Hannover (DE); Rohardt, Andreas, 29320 Hermannsburg (DE); Brossette, Dr. Tatjana, 40589 Düsseldorf (DE); Maas, Dr. Ruth, 40589 Düsseldorf (DE)
(74) Vertreter: Held, Stephan

(57) **Zusammenfassung**

Die vorliegende Erfindung betrifft Mikroorganismen, die Cellulasen auf ihrer Oberfläche präsentieren. Entsprechende Mikroorganismen wurden mit Hilfe von entsprechenden Plasmiden hergestellt, die einen Abschnitt, umfassend ein Signalpeptid, eine heterologe Cellulase, optional eine Proteaseerkennungsstelle, einen Transmembranlinker sowie eine Transporterdomäne eines Autotransporters oder eine Variante davon codieren. Solche Mikroorganismen konnten vorteilhaft bei der Umwandlung von Cellulose in Cellobiose und/oder Glucose eingesetzt werden. Es war ebenfalls möglich, die Mikroorganismen nach der Umsetzung von einfachen Substraten aus der Reaktionsmischung zurückzugewinnen. Zudem konnte eine Kombination verschiedener Mikroorganismen, die mit Exocellulasen, Endocellulasen und beta-Glucosidasen besetzt waren, zur Herstellung von Glucose aus Cellulose oder Holz eingesetzt werden.

## Beschreibung

Die Erfindung betrifft ein Nukleinsäuremotekül, umfassend die folgenden Bestandteile: (1) einen Abschnitt, der ein Signalpeptid codiert (2) einen Abschnitt, der eine heterologe Cellulase codierte (3) optional einen Abschnitt, der eine Protease-Erkennungsstelle codiert, (4) einen Abschnitt, der einen Transmembranlinker codiert, und (5) einen Abschnitt, der eine Transporterdomane eines Autotransporters oder einer Variante davon codiert.

Weltere Aspekte der vorliegenden Erfindung betreffen Polypeptide, die durch ein wie vorstehend beschriebenes Nuklünsäuremolekül codiert werden, Mikroorganismen, die an ihrer Oberfläche entsprechende Polypeptide präsentieren, sowie aus solchen Mikroorganismen erhämiche Membranfraktionen. Die vodiegeride Erfindung betrifft ebmfälls ein Verfahren zur Herstellung von Reaktionsprodukten, insbesondere von Cellulosereaktionsprodukten, unter Verwendung der Mikroorganismen oder Membranfraktionen, sowie die Verwendung der Mikroorganismen und oder Membranpräparate zur Herstellung von Produkten einer Reaktion, die durch eine Cellulase katalysiert wird.

Cellulasen sind Enzyme, die in der Lage sind, Cellulose zu threm Grundbaustein beta-Glucose abzubauen. Cellulasen werden nur von einem kleinen Teil der Pflanzen-abbauenden Organismen, wie beispielsweise speziellen Bakterien und Flagellaten, sowie von Holzabbauenden Mikroorganismen, wie Pilzen, gebildet. Mit Hilfe dieser Enzyme sind die entsprechenden Mikroorganismen in der Lage, Cellulose, die in der Regel auch aus 3000 bis 15000 Glucosemolekülen besteht, in Abbauprodukte bzw. schlussendlich in Glucose zu spalten und metabolitisch zu verwerten. Cellulasen umfassen insbesondere drei Enzymtypen, deren Zusammenwirken eine rationelle Verdauung der Cellulosemoleküle ermöglicht:
1. Endocellulasen (Enzymklasse EC 3.2.1.4): Diese Enzyme spalten Cellulose in größere Abschnitte und können im Gegensatz zu den anderen Cellulasen auch innerhalb von Celluloseketten arbeiten. Insbesondere können Endocellulasen in sogenannten amorphen Bereichen, in denen die Cellulosemoleküle ungeordnet zueinander vorliegen, aktiv werden. Dadurch erzeugen die Enzyme eine größere Anzahl von Kettenenden.
2. Exocellulsen (EC 3.2.1.91): Diese Enzyme spalten Cellulose nur von deren Ende, wobei sie die Celluloseketten kontinuierlich verkürzen. Durch die Reaktion der Exocellulasen werden typischerweise Cellobioseeinheiten (Disaccharide), aber auch Glucose, Cellotriose oder Cellutetraose von der Cellulosekette abgespalten.
3. Celloblase oder beta-Glucosidase (EC 3.2.1.21): Diese Gruppe von Enzyme spaltet die betaglycosidlsche Bindung zwischen den beiden Glucosemolekülen der Cellobiose und stellt damit Glucose für weitere Stoffwechseiprozesse bereit.

Die genannten drei Enzymtypen sind jwells für sich allein in der Lage cellulosische Biomasse angreifen. Es ist jedoch davon auszugehen, dass sich eine Kombination der genannten Enzymtypen in hohem Maße synergistisch verhält da sie unterschiedliche natürliche Struktureinhelten der cellulosischen Biomasse angreifen. Insbesondere ist bei Einsatz einer Cellobioase zu erwarten, dass der Abbau der von den Endo- und Exocellulasen bereitgestellten Cellobiosen, Cellotriosen und weiteren höheren Cellooligosacchariden zu Glukose zu einer Gleichgewichtsverschiebung der enzymatisch katalysierten Reaktionen führt und somit eine effektivere Umsetzung gefördert wird.

Cellulose und Hernicellulose sind Hauptbestandteile von Holz und vielen Pflanzen und stellen damit einen leicht verfügbaren Rahstoff dar. Eine Problematik bei der Veredelung des Materials besteht jedoch in der Schwierigkeit seiner Spaltung, beispielsweise zu Glucose, was seiner extensiven Nutzung zur Gewinnung von Bioethanol entgegensteht. Im Stand der Technik werden derzeit zur Zersetzung von Cellulose zu Glucose aufgereinigte Enzyme, die in der Regel aus Pilzkulturen wie *Trichoderma reesei oder* C *acetobulylicum* (vgl. Shah et al., App. Biochem. Blotech. (1991), vol 18/29, 99 - 106) isoliert werden müssen, eingesetzt. Die Aufreinigung der einzelnen Enzyme und Mischung eines effektiven Enzymcocktails vor Einsatz in der Zersetzung der cellulosischen Biomasse stellt dabei im heutigen Stand der Technik einen signifikanten Kostenfaktor dar. Zudem ist es im Stand der Technik nicht möglich, die entsprechenden freien Enzyme aus dem erhaltenen Produkt zurückzugewinnen. Es besteht daher ein Bedarf nach einer Konfektionierungsform von Cellulasen, die zum einen einen vereinfachten Herstellungs- und Mischungsprozess ermöglicht und zum anderen eine Rückgewinnung der Enzyme nach deren Verwendung in einfacher Weise zulässt.

Eine Aufgabe der vorliegenden Erfindung besteht daher darin, ein Agens mit einer Cellulaseakivität bereitzustellen, wobei das Agens nicht nur für Substratmoleküle leicht zugänglich ist, sondern sich auch auf einfache Weise amplifizieren, recycein und regenerieren lässt. Dadurch sollen mehrere Katalyseschritte ermöglicht werden, ohne dass es erforderlich ist, wiederholt neues Agens herzustellen. Eine weitere Aufgabe der vorliegenden Erfindung besteht darln, ein Agens bereitzustellen, das im Hinblick auf seine Genetik, Thermostabilität, Lagerfähigkeit und Stabilität günstige Eigenschaften aufweist.

Die Erfinder haben Überraschenderweise festgestellt, dass unter Verwendung eines Autotransportersystems Cellulasen an der Oberfläche von Mikroorganismen exprimiert werden können und hinsichttich der Reaktionsbedingungen für die Spaltung von Cellulose und der späteren Abtrennung der erhaltenen Produkte ausreichend stabil sind. Zudem wurde Überraschenderweise festgestellt, dass Mikroorganismen, die auf ihrer Oberfläcne entsprechende Cellulasen tragen, inkubiert werden können und in verschiedenen Lösungen und Puffern unter verschiedenen Bedingungen längere Zeit stabil bleiben.

Das Autodisplay stellt ein elegantes Werkzeug dar, um rekombinante Proteine an der Bakterienoberfläche zu präsentieren. Dieses Expressionssystem basiert auf dem Sekretionsmechanismus der Proteinfamille der Autotransporter, die zum Typ-V-Sekretionssystem gehören.

Bel gramnegativen Bakterien bildete sich der Autotransparter-Weg sowohl für den Transport. von Proteinen zur Zelioberfläche als auch für die Sekretion von Proteinen in den Extrazellularraum heraus (Jose und Meyer, 2007). Die Autotransporter-Proteine werden als Vorläuferproteine synthetisiert, die alle strukturelien Voraussetzungen für den Transport zur Zelloberfläche erfüllen (Jose, 2006). Sie werden mit einem N-terminalen Signalpeptid synthetisiert, welches für den Sec-Weg typisch ist, der das Durchqueren der inneren Membran ermöglicht. Im Periplasma angelangt, faltet sich der C-terminale Teil des Vorläufers nach Abspaltung des Signalpeptids als eine parenartige Struktur, ein sogenanntes B-Barrel, in die äußere Membran hinein. Durch diese Pore hindurch wird die N-terminal gebunden Passagierdomäne zur Oberfläche transloziert (Jose et al., 2002). Dort kann sie - entweder autoproteolytisch oder durch eine zusätzliche Protease - abgespalten werden oder über die Transporterdomäne an der Zellhülle verankert bleiben.

Ein Ersetzen des natürlichen Passagiers durch ein rekombinantes Protein führt zu dessen Oberflächentranstokation Hierfür muss mit gentechnischen Verfahren ein nicht-natürlicher Vorläufer konstruiert werden, der aus einem Signalpeptid, dem rekombinanten Passgier, dem ß-Barrel und einer Linkerregion dazwischen, die für einen uneingeschränkten Zugang zur Oberfläche erforderlich ist, besteht. Auf diese Welse ist bereits der AIDA-1-Autotransporter mit Erfolg für eine effiziente Oberflächenpräsentation verschiedener Passagierdomänen verwendet worden (Henderson et al., 2004). Bei dem Autodisplay-System wurde eine Selbst-Assoziation von Untereinheiten an ein aktives Enzym beobachtet, beispielweise bei dem dimeren Enzym Sorbitol-Dehydrogenase (Jose, 2002; Jose und von Schwichow, 2004).

Im Besonderen ist die Autodisplay-Technologie ein Expressionsverfahren für bestimmte Proteine an der Oberfläche der äußeren Membran von E. coli und anderen gramnegativen Bakterien, wobei das Autodisplay-System auf dem natürlichen Sekretionsmechanismus der Autotransporterproteine basiert (A. Banerjee et al. (2002)). Dabei kann der Transport des rekombinanten Pasmgierproteins einfach durch Einbringen einer codierenden Sequenz im Leseraster zwischen das Signalpeptid und die translozierende Domäne des Autodisplay-Vektors unter Verwendung von üblichen gentechnischen Verfahren erfolgen. Das Signalpeptid kann von einer Untereinheit des Cholera-Toxins gewonnen werden, und es kann mit einem nichtnatürlichen Promotor kombiniert werden. Folglich wird das Passagierprotein, das eine Translokation durch die äußere Membran erfahren soll, als rekombinantes Fusionsprotein mit einem anderen Protein, Autotransporter genannt, an der äußeren Membran von E. coli exprimiert (AIDA-I) (Jose, 2006). Der C-terminale Teil des Autotransporterproteins bildet eine porenartige Struktur (ß-Barrel) innerhalb der äußeren Membran von E. coll. Diese porenartige Struktur ermöglitcht eine Translokation des rekombinanten Passierproteins an die Oberfläche der äußeren Membran von E. coli (Jöse, 1995, 2006, 2007).

Das Konzept des Autodisplay-Systems, das zuerst von Jose et al. (1995) beschrieben wurde, ist seit mehr als 15 lahren bekannt. In der Zwischenzeit konnte gezeigt werden, dass sich damit Zellen mit darauf immobilisieren Nitrilasen (vgl. WO 2011/057820) und redoxaktiven Enzymen, die ihrerseits Redoxfaktoren, insbesondere NAD/NADH bzw. NADP/NADPH, regenerieren (vgl. WO 2012/025628) herstellen lassen, die die gewünschte Aktivität zeigten. Dennoch stellt die Übertragung des Autodisplay-systems auf eine neue Enzymklasse den Fachmann immer noch vor erhebliche Herausforderungen, insbesondere, da eine korrekte Faltung der Proteine in Abwesenheit der dazu teilweise notwendigen Chaperone nicht gesichert ist. Bisher wird durch den Stand der Technik die Verwendung des Autotransporter-Systems zum Immobilisieren von Cellulasen weder gelehrt noch vorgeschlagen.

Die Aufgabe der vorliegenden Erfindung wird durch die Gegenstände der unabhängigen Ansprüche gelöst. Bevorzugte Ausführungsformen könne den abhängiges Ansprüchen entnommen werden.

Die Aufgabe der vorliegenden Erfindung wird gemäß einem ersten Aspekt durch ein Nukleinsäurermolekül, umfassend
(1) einen Abschnitt, der ein Signalpeptid codiert,
(2) einen Abschnitt, der eine heterologe Cellulase oder eine Variante davon codiert,
(3) optional einen Abschnitt, der eine Protease Erkennungstelle codiert,
(4) einen Abschnitt, der einen Transmembranlinker codiert und
(5) einen Abschnitt, der eine Transporterdomäne eines Autotransporters oder eine Variante davon codiert, gelost.

Weitere Aspekte der voriegenden Erfindung betreffen Proteine bzw. Polypeptide, die durch Transkription der entsprechenden Nukleinsäuremoleküle erhältlich sind, sowie Mikroorganismen, die entsprechende Proteine enthalten und aus solchen Mikroorganismen erhältliche Membranpräparate. Ein weiterer Aspekt der vorliegenden Erfindung betrifft ein Verfahren zur Herstellung eines Reaktionsprodukts mit Hilfe von mindestens einer Cellulase, umfassend die folgenden Schritte:
(i) Bereitstellen von mindestens einem Mikroorganismus, der eine Cellulase auf einer Oberfläche präsentiert und/oder eines Membranpräparates dieses Mikroorganismus, und
(ii) Inkontsktbringen des mindestens einen Mikroorganismus und/oder des Membranpräparates mit ein oder mehreren Cellulasesubstraten unter Bedingungen, die mit der Cellulaseaktivität kompatibel sind.

Schließlich betrifft die vorliegende Erfindung auch die Verwendung der beschriebenen Mikroorganismen bzw. Membranpräparate zur Herstellung von durch Celluiasekatalyse herstellbaren Reaktionsprodukten, sowie Verfahren zur Herstellung entsprechender Mikroorganismen und Membranpräparate.

In einer bevorzugten Ausführungsform der vorliegenden Erfindung ist das Nukteinsäuremolekül mit einer Sequenz zur Expressionsregulation funktionell verbunden. In einer bevorzugten Ausführungsform verweist der Begriff "Sequenz zur Expressionsregulation", wie hier gebraucht, auf eine Nukleinsäuresequenz, welche die Höhe der Expression eines Nukleinsäuremoleküls, vorzugsweise strangabwärts von der Sequenz zur Expressionsregulation regulieren kann. Die Sequenz zur Expressionsregulation kann beispielsweise ein Promotor sein. Der Fachmann ist mit zur Expressionsregulation geeigneten sequenzen und Verfahren zum funktionellen Verbinden dieser Sequenzen mit einer Nukleinsäuremolekül vertraut. Im Zusammenhang mit der vorliegenden Erfindung sind insbesondere Expressionsregulationssequenzen bevorzugt, die für Expressionsregulatoren kodieren, die sich mit Hilfe der Induktoren Isopropylthioglucopyrariosid (IPTG) oder Arabinose, insbesondere L-Arabinose, aktivieren lassen.

In einer bevorzugten Ausführurigsform der vorliegehden Erfindung ist das Nukleinsäuremolekül Teil eines rekombinanten Plasmids.

In einer bevorzugten Ausführungsform der vorliegenden Erfindung verweist der Begriff "heterolog", wie hier gebraucht, auf ein Nukleinsauremolekül, das unter Verwendung gentechnischer Verfahren konstruiert wurde, beispielsweise durch Zusammenfügen einer Sequenz zur Expressionsregulation und einer zu exprimierenden Sequenz, die normalerweise nicht dieser Sequenz zur Expressionsregulation untersteht, oder durch Verwenden einer Sequenz, die bezüglich der ürsprünglichen Sequenz eine Punktmutation aufweist. Wenn auch nur ein Abschnitt eines Konstrukts als heterolog bezeichnet wird, impliziert das folglich, dass das gesamte Konstrukt hetemlog ist. Der Fachmann ist mit gentechnischen Verfahren vertraut. In einer weiteren bevorzugten Ausführungsform verweist der Begriff "heterolg", wie hier gebraucht, auf ein Polypeptid codierendes Nukleinsäuremolekül, das mit einer Sequenz zur Expressionsregulation zusömmengefügt ist, und/oder eine fusionierte sequenz, die von einen anderen Organismus als die Sequenz zur Expressionsregulation stammt und zu exprimierten ist. In einer weiteren bevorzugten Ausführungsform bedeutet der Begriff "heterolog", wie hier im Zusammenhang mit eines Cellulase Polypeptid gebraucht, dass der Abschnitt des Nukleinsäuremoteküls, das die Cellulase codiert, von einem anderen Organismus als mindestens ein weiterer Abschnitt, wie etwa die Transporterdomäne oder die Sequenz zur Expressionsregulation oder der Transmembranlinker, gewonnen oder genommen wurde. Beispielsweise ist die Cellulase heterolog, wenn sie aus *Bacillus subtilis* gewonnen wurde, alle anderen Sequenzen jedoch von *E. coli*, gewonnen wurden. In einer weiteren bevorzugten Ausführungsform bedeutet der Begriff "heterolog", wie hier gebraucht, dass die als heterolog bezeichnete Nukleinsäuresequenz von einem Organismus stammt, der von dem Wirt oder vorgesehenen Wirt, der zur Expression oder Vermehrung dieser Nukleinsäuresquenz verwendet wird, vershieden ist.

In einer besonders bevorzugten Ausführungsform umfasst das Nukleinsäuremolekül eine der Sequenzen SEQ ID NO:1, SEQ ID NO:3 oder SEQ ID NO:5 oder Varianten davon.

In einer bevorzugten Ausführungsform der vorliegenden Erfindung verweist der Begriff "Signalpeptid", wie hier gebraucht, auf eine Sequenz von Aminosäuren, vorzugsweise am N-Terminus eines Polypeptids, die bewirkt, dass das Polypeptid, wenn es Im Zytosol elner Wirtszelle exprimiert wird, zu einem bestimmten Kompartiment der Zelle, vorzugsweise einem vom zytosol verschiedenen Kompartiment, transloziert wird. In einer besonders bevorzugten Ausführungsform ist die Wirtszelle eine gramnegative Bakterienzelle und das Signalpeptid bewirkt, dass das entstehende oder fertige Polypeptid in das Periplasma oder die äußere Membran der gramnegativen Bakterienzelle transloziert wird.

In einer weiteren bevorzugten Ausführungsform der vorliegenden Erfindung verweist der Begriff "Protease-Erkennungsstelle", wie hier gebraucht, auf ein bestimmtes Aminosäurrensequenzmotiv In einem Polypeptid, wobei dieses Sequenzmotiv von besagter Protease in spezifischer Weise erkannt wird, derart, dass sie anbindet und das Polypeptid spaltet.

Im Rahmen der vorliegenden, Erfindung ist es ebenso bevorzugt, wenn der Begriff "Transmembranlinker", wie hier gebraucht, auf einen flexiblen Polypeptidabschnitt verweist, der zur Verbindung der Autotransporterdomäre mit dem redoxfaktor-regenerierenden Polypeptid dient, jedoch flexibel genug ist, um ein unabhängiges Falten und/oder Transportieren des redoxfaktor-regenerierenden Polypeptids zu ermöglichen.

In einer bevorzugten Ausführungsform der vorliegenden Erfindung verweist der Begriff "Transporterdomäne eines Autotransporters" auf eine Domäne, die verwendet werden kann, um das Expressionsprodukt des Nukleinsäuremoleküls zu erhalten, wenn es im Inneren der Zelle, vorzugsweise im bakteriellen Zytoplasma, ribosomal synthetisiert und zu der äußeren Membran der Zelle, vorzugsweise zu der Seite der äußeren Membran, die der Zellumgebung ausgesetzt ist, transloziert wird. In einer besonders bevorzugten Ausführungsform bewirkt die Transporterdomäne, dass sich das besagte Expressionsprodukt an der Außenfläche der äußeren Membran befindet. In einer bevorzugten Ausführungsform der vorliegenden Erfindung ist die Transporterdomäne eines Autotransporters ein Protein, dass sich an der äußeren Membran der, Zelle befindet, und der eine Cellulase codierende Abschnitt ist Teil einer Domäne, Schleife oder elnes anderen Teils der Transporterdomäne oder damit fusioniert, so dass die Cellulase an der Oberfläche der Zelle präsentiert wird. In einer weiteren bevorzugten Ausführungsform der vorliegenden Erfindung ist die Transporterdomäne eines Autotransportes ein Protein eines Systems, das verwendet werden kann, um Polypeptide an der Oberfläche einer Zelle zu präsentieren.

In einer bevorzugten Ausführungsform sind Varianten von Aminosäuren oder Nukleinsäuresequenzen, auf die in der vorliegenden Anmeldung explizit, beispielsweise durch den Namen oder die Hinterlegungsnummer oder gar durch den Begriff "Variante von", der implizit verwiesen wird, beispielsweise durch eine Beschreibung der Funktion, im Rahmen der vorliegenden Erfindung. In einer bevorzugten Ausführungsform umfasst der Begriff "Variante", wie hier gebraucht, Aminosäure- bzw. Nukleinäuresequenzen, die zu 60, 70, 75, 80, 85, 90. 92, 94, 95, 96, 97, 98 oder 99 % mit der als Referenz dienenden Aminosäure identisch sind und unter vergleichbaren Bedingungen eine ähnliche Cellulase-Aktivität aufweisen. In einer bevorzugten Ausführungsform umfasst der Begriff "Variante" im Hinblick auf eine Aminosäuresequenz solche Aminosäuresequenzen, die einen konservativen Aminosäureaustausch oder mehrere konservative Aminosäurenaustausche in Bezug auf die Referenzsequenz aufweisen. In einer bevorzugten Ausführungsform umfassen die Begriffe "Varianten" einer Aminosäuresequenz oder Nukleinsäuresequenz aktive Abschnitte und/oder. Fragmente der Aminosäuresequenz bzw. Nukleinsäuresequenz. In einer bevorzugten Ausführungsform verweist der Begriff "aktiver Abschnitt", wie hier gebraucht, auf eine Aminosäuresequenz oder eine Nukleinsäuresequenz, die kürzer als die Aminosäure- oder Nukleinsäuresequenz voller Länge ist, jedoch zumindest einen Teil ihrer wesentlichen biologischen Aktivität z.B. als eine Cellulase, bewahrt In einer bevorzugten Ausführungsform umfasst der Begriff "Variante" einer Nukleinsäure die Nukleinsäuren des komplementären Strangs, die - vorzugsweise unter stringenten Bedingungen - mit der Referenznukleinsäure hybridisieren. Die Stringenz von Hybridisierungsreaktionen ist von einem Durchschnittfachmann ohne Weiteres bestimmbar und ist üblicherweise eine empirische Berechnung in Abhängigkeit von der Sondenlänge, der Waschtemperatur und der Salzkonzentration. Im Allgemeinen erfordern längere Sonden höhere Temperaturen für ein einwandfreies Anneallng, während kürzere Sonden niedrigere Temperaturen benötigen. Die Hybridisierung hängt Im Allgemeinen von der Fähigkelt der denaturierten DNA zur Renaturierung ab, wenn komplementäre Stränge in einer Umgebung vorliegen, deren Temperatur niedriger als die Schmelztemperatur der Stränge ist. Je höher der Grad der angestrebten Homologle zwischen der Sonde und einer hybridisierbaren Sequenz ist, desto höher ist die relative Temperatur, die angewandt werden kann. Dadurch ergibt sich, dass höhere relative Temperaturen tendenziell die Reaktionsbedingungen stringenter machen, während niedrigere Temperaturen diese Stringenz verringem. Weitere Einzelheiten und eine Erläuterung der Stringenz von Hybridisierungsreuktionen können Ausubel et al. (1995) entnommen werden. In einer weiteren bevorzugten Ausführungsform verweist der Begriff "Variante" einer Nukleinsäure oder Aminosäure auf eine Nukleinsäure bzw. Aminosäure, die, zumindest bis zu einem gewissen Grade, die gleiche biologische Aktivität und/oder Funktion wie die Referenz-Nukleinsäure oder - Amlnosäure hat. In einer bevorzugten Ausführungsform verweist der Begriff "Variante" einer Nukleinsäuresequenz, wie hier gebraucht, auf eine andere Nukleinsäuresequenz, welche eine der Referenz-Aminosäuresequenz gleichende Aminosäuresequenz codiert.

In einer bevorzugten Ausführungsform verweist der Begriff "Cellulase" auf ein Enzym der Klasse EC 3.2.1.4, EC 3.2.1.91 oder EC 3.2.1.21. In einer weiteren bevorzugten Ausführungsform ist die Cellulase eine Cellulase aus *Bäcillus subtilis* oder *Clostridium thermocellum.* Die Cellulase kann eine Endo-, Exo- oder Exocellulase oder eine beta-Glucosidase sein. Im Rahmen der vorliegenden Erfindung ist es jedoch bevorzugt, wenn es sich um eine Endocellulase aus *bacillus subtilis,* eine Exocellulase aus *Costridium thermocellum* oder eine beta-Glucosidase aus *Costridium thermocellum* handelt. Besonders bevorzugt handelt es sich um ein Enzym mit einer Sequenz gemäß SEQ ID NO:2, SEQ ID NO:4 oder SEQ ID NO:6 oder Varianten davon.

Vorzugsweise ist die Cellulase mit einer Transporterdomäne eines Autotransporters füsloniert

Die Transporterdomäne des Autotransporters gemäß der Erfindung kann eine beliebige Transporterdomäne eines Autotransporters sein und ist vorzugsweise imstande, eine B-Barrel-Struktur zu bilden. Eine ausführliche Beschreibung von B-Barrel-Strukturen und bevorzugte Belspiele für B-Barrel-Autotransporter sind in WO 97/35 022 offenbart und durch die Bezugnahme Bestandteil der vorliegenden Beschreibung. Henderson et al. (2004) beschreiben Autotransporteproteine mit geelgneten Autotransporterdomärien (eine Zusammenfassung kann der Tabelle 1 von Henderson et al., 2004, entnommen werden), Die Offenbarung von Henderson et al. (2004) ist durch die Bezugnahme Bestandteil der vorliegenden Beschreibung. Beispielsweise kann die Transporterdomäne des Autotransporters ausgewählt werden aus: Ssp (P09489, S. marcescens), Ssp-h1 (BAA33455, S. marcescens), Ssp-h2 (BAA11383, S. marcescens), PspA (BAA36466, P. fluorescens), PspB (BAA36467, P. fluorescens), Ssa1 (AAA80490, P. haemolytica), SphB1 (CAC44081, B. pertussis), AspA/NaIP (AAN71715, N. meningltidis), VacA [Q48247, H. pylori), AIDA-I (Q03155, E. coli), IcsA (AAA26547, S. flexneri), MisL (AAD16954, S. enterica), TibA (AAD41751, E. coli), Ag43 (P39180, E. coli), ShdA (AAD25110, S. enterica), AutA (CAB89117, N. meningitidis), Tsh (I54632, E. coli), SepA (CAC05786, S. flexneri), EspC (AAC44731, E. coli), EspP (CAA66144, E. coli), Pet (AAC26634, E. coli), Pic (AAD23953, E. coli), SigA (AAF67320, S. flexneri), Sat (AAG30168, E. coli), Vat (AAO21903, E. coli), EpeA (AAL18821, E. coli), EatA (AA017297, E coli), Espl (CAC39286, E. coli), EaaA (AAF63237, E. coli), EaaC (AAF63036, E. coli), Pertactin (P14283, B. pertussis), BrkA {AAA51646, B. pertussis), Tef (AAQ82668, B. pertussis), Vag8 (AAC31247, B. pertussis), PmpD (084818, C. trachomatis), Pmp20 (Q9Z812, C. pneumoniae), Pmp21 (Q9Z6U5, C. pneumoniae), IgA1 protease (NP_283693, N. meningitidis), App (CAC14670, N. meningitidis) Iga1 protease (P45386, H. influenzae), Hap (P45387, H. influenzae), rOmpA (P15921, R. rickettsii), rOmpB (Q53047, R. rickeltsii), ApeE (AAC38796, S. enterica), EstA (AAB61674, P. aeruginosa), Llp-1 (P40601, X. luminescens), McaP (AAP97134, M. catarrhalis), BabA (AAC38081, H. pylori), SabA (AAD06240, H. pylori), AlpA (CAB05386, H. pylori), Aae (AAP21063, A. actinomycetemcomitans), NanB (AAG35309, P. haemolytica) und Varianten dieser Autotransporter. Für jedes der beispielhaften Autotransporterproteine sind in Klammern Beispiele für geeignete Genbank-Hinteriegungsnummern und Spezies angegeben, von denen der Autotransporters erhalten werden kann. Im Rahmen der vorliegenden Erfindung ist die Transporterdomäne des Autotransporters vorzugsweise ausgewählt aus der Gruppe, umfassend Ssp, Ssp-h1, Ssp-h2, PspA, PspB, Ssa1, SphB1) AspA/NaIP, VacA, AIDA-I, IcsA, MisL, TibA, Ag43, ShdA, AutA, Tsh, SepA, EspC, EspP, Pet, Pic, SigA, Sat, Vat, EpeA, EatA, Espl, EaaA, EaaC, Pertactin, BrkA, Tef, Vag8, PmpD, Pmp20, Pump21, AgA1 Protease, App, Hap, rOmpA, rOmpB, ApeE, EstA, Up-1, McaP, BabA, SabA, AlpA, Aae, NanB und Varianten davon. In einer basonders bevorzugten Ausführungsform ist die Transporterdomäne eine Transporterdomäne des Autodisplay-Systems, auch als Autotransporter-Weg bezeichnet, des AIDA-I-Typs gramnegativen Bakbertenzellen, insbesondere von E. coll oder eine Variante davon, wie beispielsweise jene, die von Niewert et al. (2001) beschrieben wurden.

Varianten der oben angegebenen Autotransportersequenzen können beispielsweise durch Verändem der Aminosäuresequenz in den Schleifenstrukturen des B-Barrels, die nicht zu den Transmembranabschnitten gehören, erhalten werden. Optional können die für die Obeflächenschleifen codierenden Nukleinsäuren vollständig deletiert werden. AuBerdem können innerhalb der amphipathischen ß-Faltblattstrukturen konservative Aminosäurenaustausche, d. h. der Austausch einer hydrophilen gegen eine andere hydrophile Aminosäure und/oder der Austausch einer hydrophoben gegen eine andere hydrophobe Aminosäure, vorgenommen werden. Vorzugsweise hat eine Variante auf Aminosäureebene eine Sequenzidentität von mindestens 70 %, mindestens 80 %, mindestens 90 %, mindestens 95 % oder mindestens 98 % mit der entsprechenden natürlich vorkommenden Sequenz der Autotransporterdornäne, insbesondere im Bereich der β-Faltblattstrukturen.

Wie vorstehend beschrieben, wird die Aufgabe der vorliegenden Erfindung in einem Aspekt durch einen Mikroorganismus gelöst, der an seiner Oberfläche ein Polypeptid, wie vorstehend beschrieben, exprimiert oder unter Verwendung eines Nukleinsäuremoleküls, wie vorstehend beschrieben, transformiert worden ist.

In einer bevorzugten Ausführungsform verweist der Begriff "Mikroorganismus", der austauschbar gegen den Begriff "Wirtszelle" gebraucht wird, wie hier gebraucht, auf einen Mikroorganismus, der imstande ist, ein Polypeptid zu exprimieren. Ein solcher Mikroorganismus kann auch als "Ganzzell-Katalysator oder -Blokatalysator" bezeichnet werden. In einer weiteren bevorzugten Ausführungsform ist der Mikroorganismus oder die Wirtszeille eine prokaryotische Zelle, vorzugsweise eine gramnegative Bakterienzelle, ganz besonders bevorzugt eine E. coli-Zelle, In einer weiteren bevorzugten Ausführungsform ist der Mikroorganismus oder die Wirtszelle eine eukaryobsche Zelle oder eine Spore eines Prokaryoten.

Eine Bakterienzelle umfasst eine Reihe von Kompartimenten, die durch hydrophobe Membranen voneinander getrennt sind. Eine grampositive Bakterienzelle weist eine Plasmamembran auf, die das Zytosol, das Innere der Zelle, begrenzt. Die Plasmamembran ist von einer Peptidoglykanschicht umgeben. Gramnegattve Baktarien hingegen besitzen zusätzlich zu der Plasmamembran eine weltere Membran, die als äußere Membran bezeichnet wird. Der Begriff "Oberfläche", wie hier gebraucht, verweist vorzugsweise auf eine Schickt des Mikroorganismus, die der Umgebung ausgesetzt ist, wobei die Umgebung belspielsweise das flüssige Kulturmedium ist, das zum Züchten des Mikroorganismus von Interesse verwendet wird. In einer ganz besonders bevorzugten Ausführungsform wird ein Polypeptid gemäß der vorliegenden Erfindung an der AuBenseite der äuBeren Membran einer gramregativen Bakterienzelle exprimiert. In elner bevorzugten Ausführungsform wird ein Polypeptid gemäß der vorliegenden Erfindung an der Innenseite der äußeren Membran einer gramnegativen Bakterienzelle exprimiert.. In einer weiteren bevorzugten Ausführungsform wird das erfindungsgemäße Polypeptid an der Außenseite eines Sphäroplasten exprimiert, wobei es sich um eine gramnegative Bakterierizelle handelt, bei der die äußere Membran worden entfernt ist. Der Fachmann ist mit Verfahren vertraut, die verwendet werden können, um Sphäroplasten herzustellen. In einer weiteren bevorzugten Ausführungsform wird das erfindungsgemäße Polypeptid an der Außenseite einer grampositiven Bakterienzelle exprimiert. So, wie die Begriffe "an der Oberfläche präsentiert" und "an der Oberfläche exprimiert" hier gebraucht werden, sind sie synonym.

Gemäß einem weiteren Aspekt wird die Aufgabe der vorliegenden Erfindung durch eine Membranfraktion gelöst, die aus dem Mikroorganismus gemäß dem vorstehende Aspekt der vorliegenden Erfindung gewonnen werden kann.

In einer bevorzugten Ausführungsform der vorliegenden Erfindung umfasst die Membranfraktion oder das Membranpräparat, wobei die beiden Begriffe austauschbar gebraucht werden, eine Cellulase gemäß dem ersten Aspekt der Erfindung, vorzugsweise in einem katalytisch aktiven Zustand. Die Begriffe "Membranfraktion" und "Membranpräparat", wie hier gebraucht, verweisen vorzugsweise auf ein Produkt, das sich in Membranbestandtellen anreichert, vorzugsweise Bestandteilen der äußeren Membran einer gramnegativen Bakterie. Der Fachmann ist mit Methodenvorschriften und Verfahren vertraut, die verwendet werden, können, um Membranpräparate herzustellen. Zum Belspiel können Bakterienzellen von einer Kultur geerntet und einer Lyse unterzogen werden, beispielsweise durch Gefrier-Auftau-Zyklen, Beschallung, Resuspension In Lysepuffer oder dergleichen, gefolgt von einer Differentiaizentrifugation, um Membrenfraktionen der Zellen zu Isolleren. In einer bevorzugten Ausführungsform der vorliegenden Erfindung ist das Membranpräparat ein Präparat der äußeren Membran, d. h. ein Präparat, bei dem die Bestandteile der äußern Membran, verglichen mit den Bestandteilen anderer Membranen und Kompartimente, wie etwa des Zytosols, der inneren Membran und des Periplasmas, angereichert sind. Der Fachmann ist mit Methodenvorschriften und Verfahren vertraut, die verwendet werden können, um die äußere Membran oder Bestandteile davon zu isolieren oder anzureichern, wie beispielsweise eine Lysozymbehandlung von Bakterienzellen und nachfolgende Zentrifugationsschritte. In einer bevorzugten Ausführungsform kann die Membranfraktion eine behandelte, Membranfraktion sein, d. h. dass der Gehalt oder die Eigenschaften der Membranfraktion verändert worden sind, beispielsweise durch Aufreinigen eines Proteinbestandteils der Membranfraktion oder durch Solubilisieren der Membranfraktionen und/oder Aufnahme von Bestandteilen der Membranfraktion in Vesikel. In einer bevorzugten AusFührungsform der vorliegenden Erfindung kann die Membranfraktion immobilisiert sein beispielsweise an der Oberfläche eines Gefäßes oder einer Säule.

Gemäß einem weiteren Aspekt wird die Aufgabe der vorliegenden Erfindung gelöst durch ein Verfahren zur Herstellung eines Reaktionsprodukts mit Hilfe von mindestens einer Cellulase, umfassend die folgenden Schritte:
(i) Bereitstellen von mindestens einem Mikroorganismus der eine cellulase auf seiner Oberfläche präsentiert, und/oder eines Membranpräparat dieses Mikroorganismus, und (ii) Inkontaktbringen des Mikroorganismus und/oder des Membranpräparat mit ein oder mehreren Cellulase Substraten unter Bedingungen, die mit der Cellulase Aktivität kompatibel sind. Der Ausdruck "präsentiert" ist im Zusammenhang mit der vorliegenden Erfindung so zu verstehen, dass die Cellulase in das den Mikroorganismus, oder die Mikroorganismen umgebende Medium hineinragt.

Das erfindungsgemäße Verfahren lässt sich besonders Vorteilhaft ausgestallen, indem das Produkt der durch die Cellulase katalysierten Reaktion ein Mono-, Di- oder Oligosaccharid ist, und indem das mindestens eine Cellulasesubstrat eine Polysaccharidquelle, besonders bevorzugt eine Cellulosequelle ist. Die Begriffe "Polysaccharidquelle" bzw. "Cellulösequelle" sind so aufzufassen, dass die Materiallen Polysaccharide bzw. Cellulose, vorzugsweise als Hauptbestandteil enthalten, aber nicht notwendigerweise ausschließlich daraus bestehen. Handelt es sich bei der Cellulösequelle um Cellulose, so können als resultierende Produkte abhängig von der eingesetzten Cellulase, Glucose, Cellobiose bzw. auf Glucose basierende Oligosaccharide gewonnen werden.

Weiterhin lässt sich das erfindungsgemäße Verfahren dadurch vorteilhaft weiter ausgeatalten, dass es einen Schritt (iii) des Zurückgewinnens des in Schritt (ii) verwendeten Mikoorganismus umfasst Das Zurückgewlnnen, kann beispielsweise durch Abzentrifugieren der Zellen von der Reaktionsmischung erfolgen.

Im Rahmen der vorliegenden Erfindung hat es sich gezeigt, dass der Schritt (ii) vorzugsweise in einem pH-Bereich von 4,5 bis 6,5, besonders bevorzugt in einem Bereich von 5,5 bis 6,5, durchgefünrt werden sollte. Für entsprechende pH-Werte weisen Cellulasen Ihre höchste Aktivität auf. Weiterhin hat es sich als zweckmäßig herausgestellt, die Temperatur während des Schritts (ii) im Bereich von 30 bis 80°C, vorzugsweise im Bereich von 50 bis 65°C, zu wählen, da die Cellulasen in diesem Temperaturbereich die nöchsten Aktivitäten aufweisen.

Werden im vorstehend geschilderten Verfahren Cellulasen, die nicht Glucose ais Endprodukt produzieren, eingesetzt, so kann es zweckmäßig sein, die entstehenden Produkte in Glucose zu überführen, da bei Anwesenheit größerer, Mengen an Cellobiose die Aktivität von Enzyme beelnträchtigt werden kann. Im Rahmen der vorliegenden Erfindung ist die Zugabe von Glucosidasen im Schritt (ii) des vorstehend beschriebenen Verfahrens daher bevorzugt Bei diesen Glucosidasen handelt es sich zweckmäßig nicht um an Mikroorganismen gebundene Enzyme, besonders bevorzugt handelt es steh um beta-Glucosidase aus Mandel.

Als Polysaccharidquelle können jegliche Polysaccharide eingesetzt werden, die von Cellulase abgebaut werden. Im Rahmen der vorliegenden Erfindung hat es sich jedoch als besonders zweckmäßig herausgestellt, wenn als Polysaccharidquelle eine Cellulosequelle eingesetzt wird.

Bei der Cellulosequelle kann es sich entweder um aufgereinigte Cellulose mit nur geringen Antellen an Fremdsubstanzen (vorzugswelse im Bereich von weniger als 80 Gew.-%, insbesondere im Bereich von weniger als 90 Gew.-%) handeln. Insbesondere kann es sich um kommunale Abwässer handeln, die direkt eingesetzt werden können oder aus denen die Cellulose mit Hilfe bekannter Verfahren zurückgewonnen und anschließend mit dem oder den Mikroorganismen umgesetzt werden kann. Ebenso können cellulosehaltige Abwässer aus Zellstoff- oder Papierfabriken als Cellulosequelle genutzt werden. Es ist jedoch auch möglich, Cellulosequelle einzusetzen, die Verunreinigungen enthalten, wie beispielsweise Holz, das vorbehandelt sein kann. Bei dem Holz kann es sich zweckmäßig um Holz, ausgewählt aus Kiefer, Lärche, Fichte, Buche, Eukalyptus, Tanne, Pappel, Pinie, Buche, Eiche, Esche, Kastanie, Birke, Kirsche, Ahorn oder Nuss handeln. Ein im Rahmen der Erfindung bevorzugtes Holz ist Pappelholz. Alternativ können aber auch cellulosehaltige Einjahrespflanzenreste, wie beispielsweise Stroh, im erfindungsgemäßen Verfahren als Polysaccharidquelle zum Einsatz kommen. Das entsprechende Holz oder die Pflanzenreste können vorbehandelt sein, beispielsweise, um enthaltene Hemicellulose zu entfernen. Die Polysaccharidquelle kann jedoch signifikante Mengen an Lignin enthalten, ebenfalls ist es möglich, Holz oder Pflanzenreste zu verwenden, das/die Hemicellulose enthält.

Im erfindungsgemäßen Verfahren kann ein Typ von Mikroorganismen, der eine bestimmte Klasse von Cellulasen auf seiner Oberfläche präsentiert, verwendet werden, es kann jedoch auch eine Mischung von Mikroorganismen zum Einsatz kommen, die verschiedene Klassen von Cellulasen auf ihrer Oberfläche präsentieren. Es ist bevorzugt wenn im Verfahren mindestens eine Mischung von mindestens einer Endo- und mindestens einer Exocellulase zum Einsatz kommt, besonders bevorzugt eine Mischung von mindestens einer Endo- und mindestens einer Exocellulase und mindestens einer beta-Glucosidase. In einer besonders bevorzugten Ausführungsform beträgt das Verhältnis von Endo- zu Exocellulase-modifizierten Mikroorganismen 25:75 bis 75:25, ganz besonders bevorzugt 25:75 bis 40:60, wobei die Mischung frei von beta-Glucosidase-modifizierten Mikroorganismen ist. In einer weiteren bevorzugten Ausführungsform wird Im Verfahren eine Mischung von mit einer Endo- und Exoclellulase und einer beta-Glucosidase-modifizierten Mikroorganismen eingesetzt, die besonders bevorzugt im Verhältnis von etwa 1:2:1 vorlegen. Die Endocellulase umfasst vorzugsweise eine Peptidsequenz SEQ ID No:2. Die Exocellulase umfasst unabhängig davon vorzugsweise eine Peptidsequenz SEQ ID No:4. Die beta-Glucosidase umfasst vorzugsweise unabhängig davon eine Peptidsequenz SEQ ID No:6.

Ein welterer Aspekt der vorliegenden Erfindung betrifft die Verwendung von Mikroorganismen, wie sie vorstehend beschrieben sind, oder Membranfraktionen, wie sie ebenfalls vorstehend beschrieben sind, zur Herstellung eines Produkts einer Reaktion, die durch eine Cellulase katalysiert wird. In einer besonders bevorzugten Ausführungsform handelt es sich bei dem Produkt um ein Abbauprodukt von Cellulose, Insbesondere um Glucose und/oder Cellobiose.

In einer weiteren bevorzugten Ausführungsform wird die Aufgabe der vorliegenden Erfindung durch ein Verfahren zur Herstellung eines Mikroorganismus gelöst, der auf seiner Oberfläche eine Cellulase präsentiert, und das die Schrltte:
(a) Einbringen der eines wie vorstehend beschriebenen Nukleinsäuremoleküls, dass eine operativ an das Nukleinsäuremolekül gebundene Expressionskontrollsequenz enthält, in einen Mikroorganismus, und optional
(b) Inkontaktbringen des Mikroorganismus mit einer die Expressionskontrolisequenz aktivierenden Substanz, umfasst.

Ebenso wird die Aufgabe der vorliegenden Erfindung durch ein Verfahren zur Herstellung eines Membranpräparats gelöst, dass neben den vorstehend beschriebenen Schritt (a) und (b) einen daran angeschlossenen Schritt des Gewinnens des Membranpräparats aus dem Mikroorganismus umfasst. Die für diesen Schritt erforderlichen Maßnahmen entsprechen den Im Vorstehenden geschliderten.

In einer weiteren bevorzugten Ausfuhrungsform wird die Aufgabe der vorllegenden Erfindung durch eine Zusammensetzung gelöst, die eine oder mehrere der vorstehend beschriebenen Mikroorganismen, die Cellulasen auf ihrer Oberfläche präsentieren bzw. ein entsprechendes Membranpräparat, enthält.

Vorliegende Erfindung wird darüber hinaus veranschaulicht durch die folgenden Figuren, die nicht einschränkend zu verstehenden Beispiele, deren weltere Merkmale, Ausrührungsformen, Geskhtspunkte und Vorteile der Erfindung entnommen werden können.

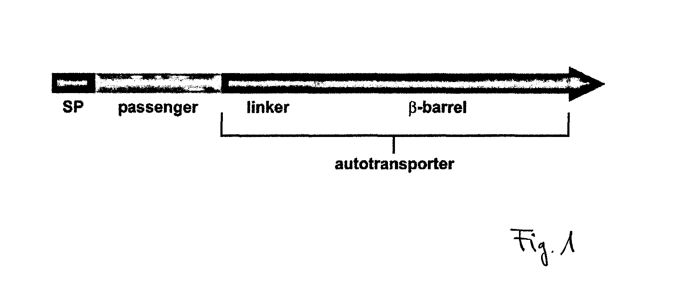

**Figur 1****:** Schematischer Aufbau der Autodisplay-Kassette. Das Signalpeptid (SP) dient dem Transport über die innere Membran. Das beta-Fass (beta-barrel) faltet sich in die äußere Membran und verankert das Konstrukt dort. Der *linker* wird zur Gewährleistung der vollständigen Translokation des Passagiers (*passenger*), in diesem Fall CipA, an die Zelloberfläche benötigt.

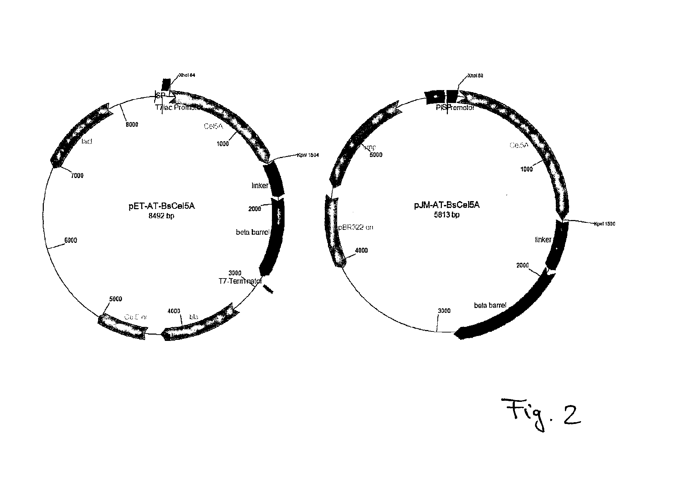

**Figur 2****:** Restriktionskarte des Plasmids zur Expression des Cel5A Im induzierbaren T7-System (links) und im konstitutiven System (rechts). Das linke Plasmid ist ein Derivat von pET-11d (Novagen) und wurde In den Stamm *E*. *coli* BL21(DE3) transformiert, das rechte Plasmid ist ein Derivat von pJM007 (Maurer et al., 1997) und wurde in *E*. *coli* BL21 transformiert.

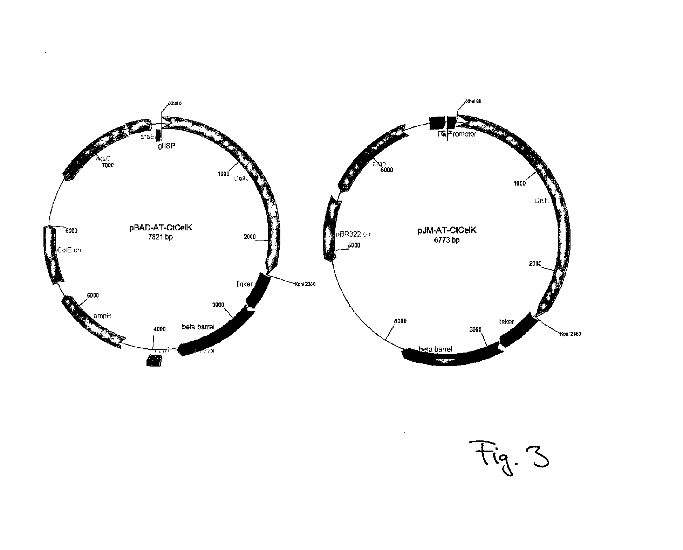

**Figur 3****:** Restriktionskarte des Plasmids zur Expression des CelK-Fusionsproteins im induzlerbaren pBAD-System (links) und im konstitutiven System (rechts). Das linke Plasmid ist ein p8AD/gIII A Derivat (Invitrogen) und wurde in *E. coli* BL21 transformiert, das rechte Plasmid ist ein Derivat von pJM007 (Maurer et al., 1997) und wurde in *E. coli* BL21 transformiert.

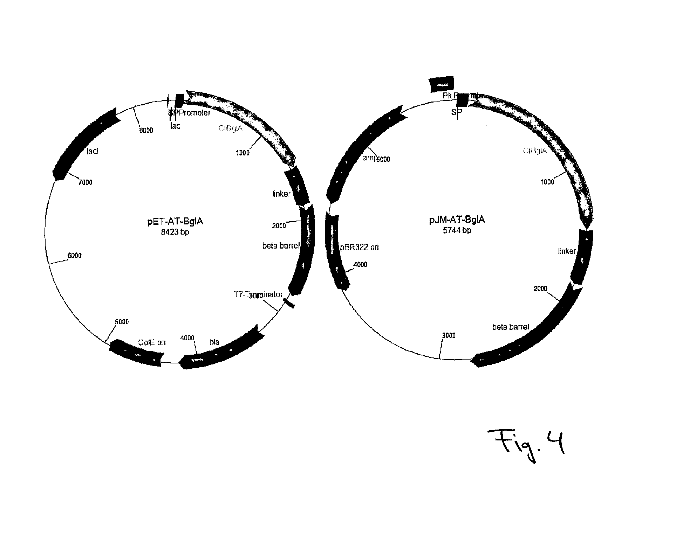

**Figur 4****:** Restriktionskarte des Plasmids zur Expression des BglA-Fusionsproteins im induzierbaren T7-System (links) und im konstitutiven System (rechts). Das linke Plasmid ist ein Derivat von pET-11d (Novagen) und wurde in den Stamm *E. coli* BL21(DE3) transformiert, das rechte Plasmid ist ein Derivat von pJM007 (Maurer et al., 1997) und wurde in *E. coli* BL21 transformiert.

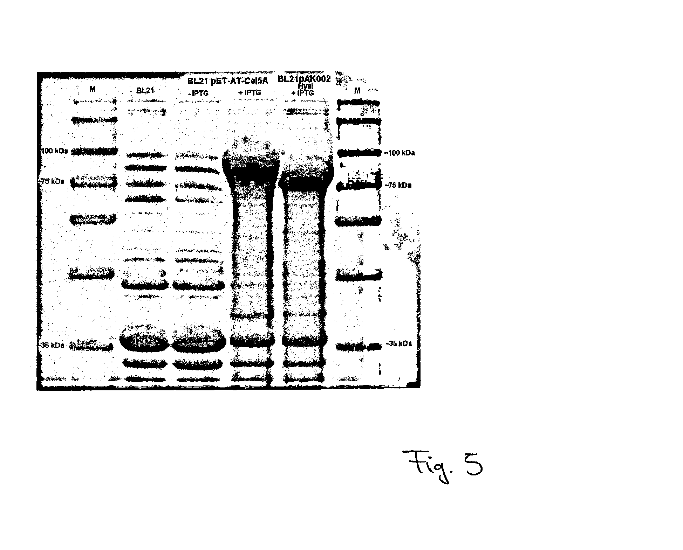

**Figur 5****:** Vergleich der Expression des Cel5A-Fusionsproteins und des Fusionsproteins der Nonsense-Kontrolle (NK) auf der Oberfläche von *E. coli* BL21(DE3). Außenmembranproteine wurden mittels differentieller Zellfraktionierung isoliert, durch SDS-PAGE (10% Acrylamid) aufgetrennt und mit Coomassie Brillant Blue gefärbt. M: Proteingrößenstandard. -/+ IPTG: nicht induziert bzw. Induziert; WT: Wirtsstamm.

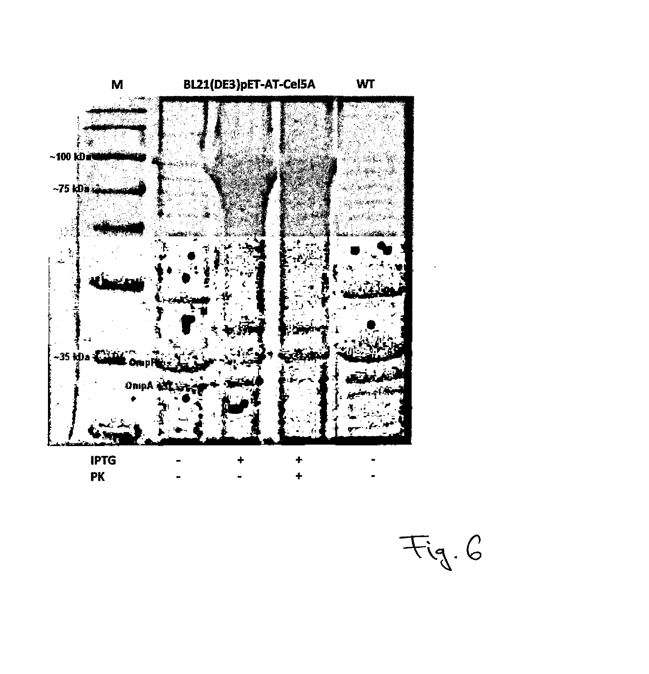

**Figur 6****:** Nachweis der Oberflächenständigkeit des Cel5A-Fusionsproteins auf *E*. *coli* BL21(DE3). Die Außenmembranproteine wurden mittels differentieller Zelifraktionierung isoliert, durch SDS-PAGE (10% Acrylamid) aufgetrennt und mit Coomassie Brilliant Blue gefärbt. M: Proteingrößenstandard; WT: Wirtsstamm; PK: Proteinase K. Der Verdau ganzer Zellen mit PK fand vor der Außenmembranprotein-Isolierung statt.

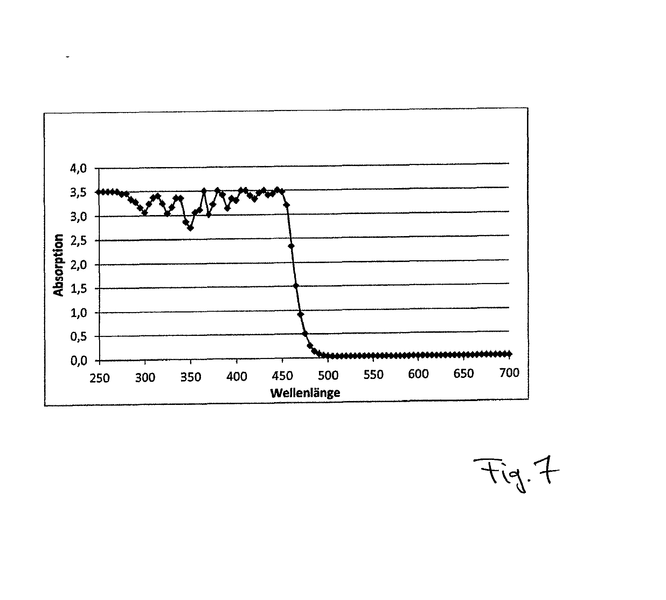

**Figur 7****:** Spektrum von p-Nitrophenolat unter CelK-Bedingungen. 5 mM p-Nitrophenol wurde in Na-Citratpuffer (pH 6) nach 10-minütiger Inkubation bei 60°C und anschlleßender Alkalisierung photometrisch vermessen.

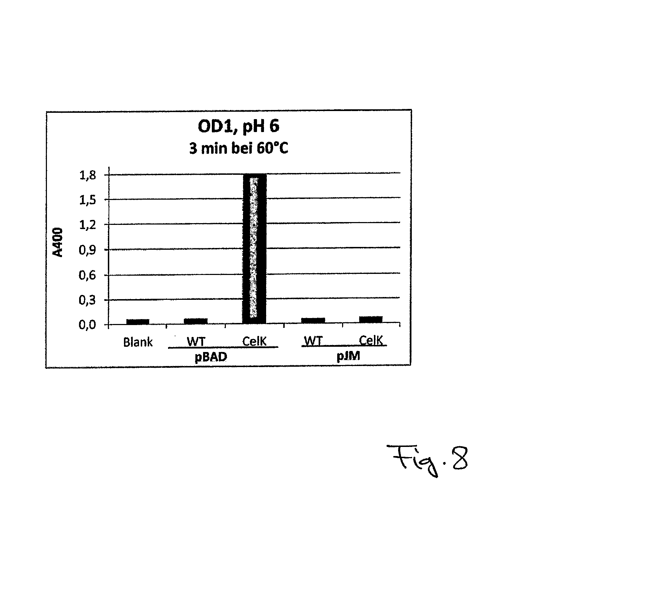

**Figur 8****:** CelK Aktivität induzierbar (pBAD) versus konstitutiv (pJM). Die Zellen wurden mit p-Nitrophenol-Cellobiosid 3 min bei 60°C und pH 6 inkubiert. Nach der Inkubation erfolgte eine Alkalisierung mit Natriumcarbonat. Das entstandene p-Nitrophenolat wurde bel 400 nm detektiert. Blank: Puffer + Substrat (ohne Zellen). WT: Wirtsstamm (ohne Plasmid).

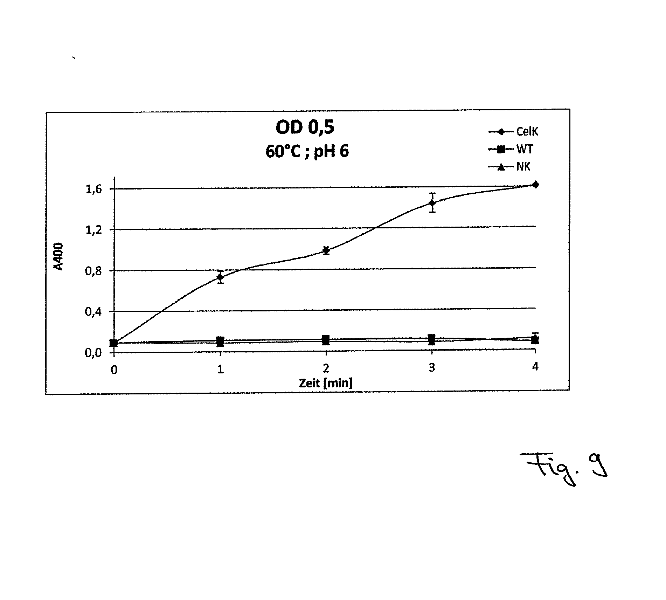

Figur 9: Nonsense Kontrolle (NK) für die CelK-Aktivität. Die Zellen wurden mit p-Nitrophenol-Cellobiosid versetzt, bei 60°C und pH 6 inkubiert. Das gebildete p-Nitrophenolat wurde nach 1, 2, 3 und 4 min bei 400 nm detektiert Eine Alkalisierung erfolgte zuvor mit Natriumcarbonat.
WT: Wirtsstamm. Abgebildet sind MW ± SD; n = 2.

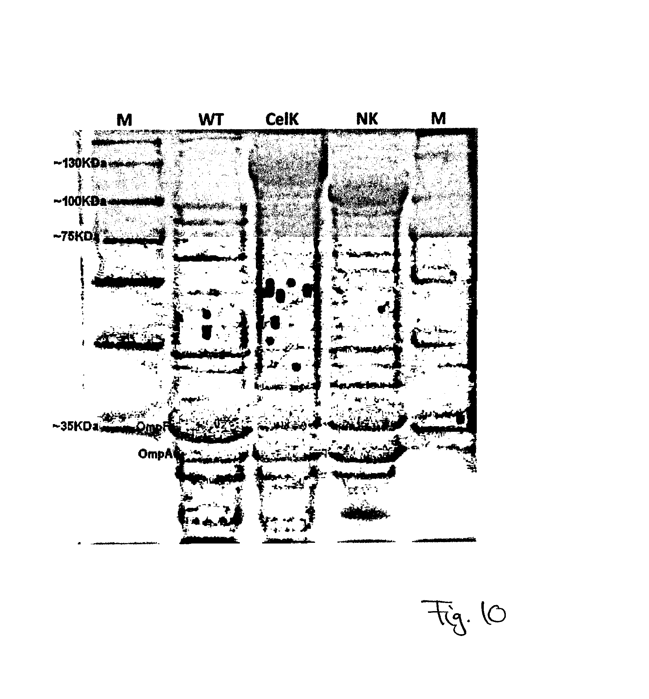

**Figur 10****:** Vergleich der Expression des CelK-Fusionsproteins und des Fusionsproteins der Nonsense-Kontrolle (NK) auf *E. coli* BL21. Außenmembranproteine wurden mittels differentieller Zellfraktionierung isoliert, durch SDS-PAGE (10% Acrylamid) aufgetrennt und mit Coomassie Brilliant Blue gefärbt. M: Proteingrößenstandard; WT: Wirtsstamm.

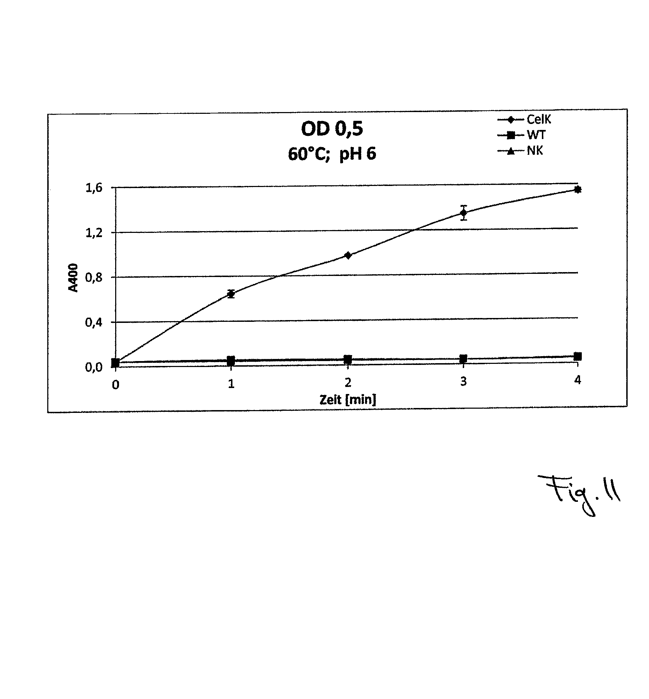

**Figur 11****:** Lagerstabilität des CelK-Stamms. Die Zellen wurden 8 Tage bei 4°C gelagert und anschließend die CelK-Aktlvität bei 60°C und pH 6 über die Zunahme des entstehenden p-Nitrophenolats bei 400 nm bestimmt. WT: Wirtsstamm; NK: Nonsense-Kontrolle. Abgebildet sind MW ± SD; n = 3.

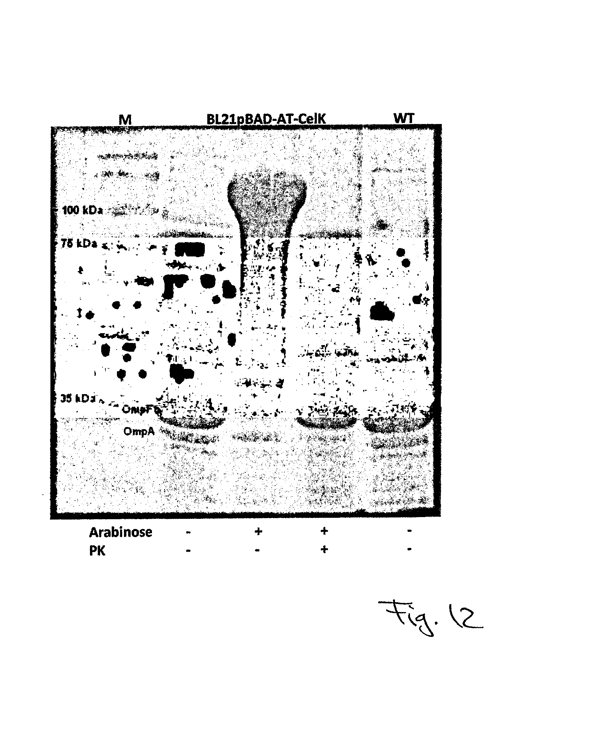

**Figur 12****:** Nachweis der Oberflächenständigkeit des CelK-Fusionsproteins auf *E*. *coli* BL21. Die Außenmembranproteine wurden mittels differentieller Zellfraktionierung isoliert, durch SDS-PAGE (10% Acrylamid) aufgetrennt und mit Coomssie Brilliant Blue gefärbt. M: Proteingrößenstandard; WT: Wirtsstamm; PK: Proteinase K. Der Verdau ganzer Zellen mit PK fand vor der Außenmembranproteln-Isolierung statt.

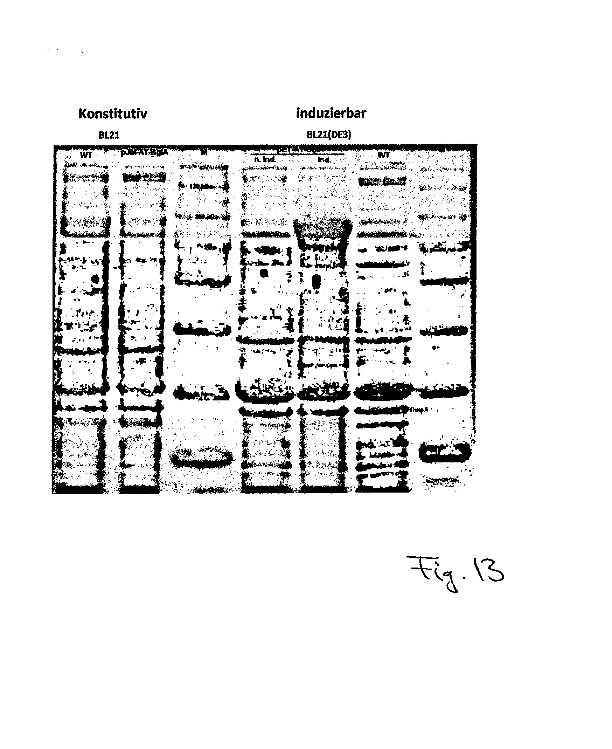

**Figur 13****:** Expression des BglA-Fusionsproteins im induzierbaren pET-System und konstitutiven pJM-System auf der Oberfläche von *E*. *coli* BL21(DE3) bzw. *E. coli* BL21. Außenmembranproteine wurden mittels differentieller zellfraktionierung isoliert, durch SDS-PAGE (10% Acrylamid) aufge-trennt und mit Coomassie Brilliant Blue gefärbt. WT: Wirsstamm; n. Ind bzw. ind.: -/+ IPTG; M: Proteingrößenstandard.

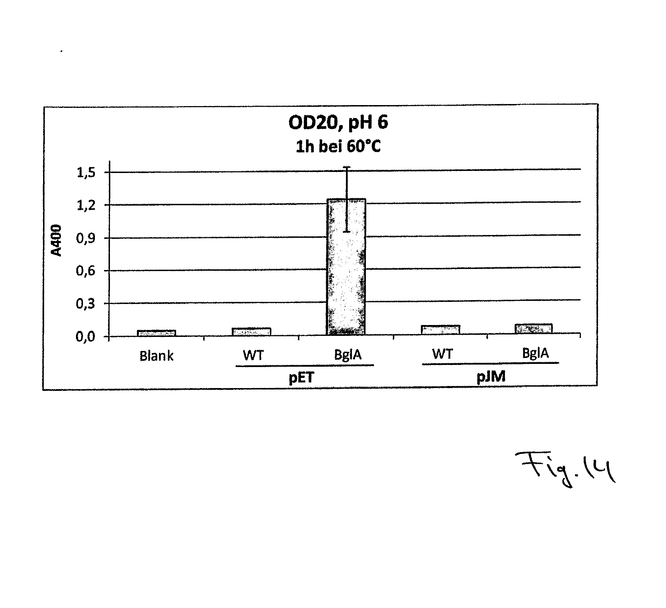

**Figur 14****:** BglA-Aktivität induzierbar (pET) versus konstitutiv (pJM). Die Zellen wurden mit p-Nitrophenol-Glucopyranosid 1 h bei 60°C und pH 6 inkubiert. Nach der Inkubation erfolgte eine Alkalisierung mit Natriumcarbonat. Das entstandene p-Nitrophenolat wurde bei 400 nm detektiert. Blank: Puffer + Substrat (ohne Zellen). WT: Wirtsstamm (ohne Plasmid), Abgebildet sind MW ± SD; n = 3.

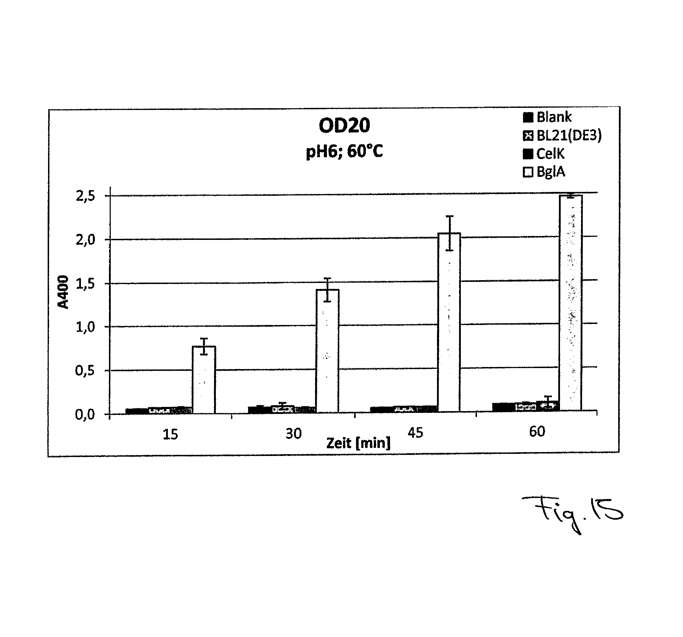

**Figur 15****:** BglA-Aktivität und Nonsense-Kontrolle (CelK). Die Zellen wurden mit p-Nitrophenol-Glucopyranosid versetzt und bei 60°C und pH 6 inkubiert. Das gebildete p-Nitrophenolat wurde nach 15, 30, 45 und 60 min bei 400 nm detektiert. Eine Alkalisierung erfolgte zuvor mit Natriumcarbonat. Als Kontrolle diente der Wirtsstamm *E*. *coli* BL21(DE3). Blank: Puffer + Substrat (ohne Zellen). Abgebildet sind MW ± SD; n = 3.

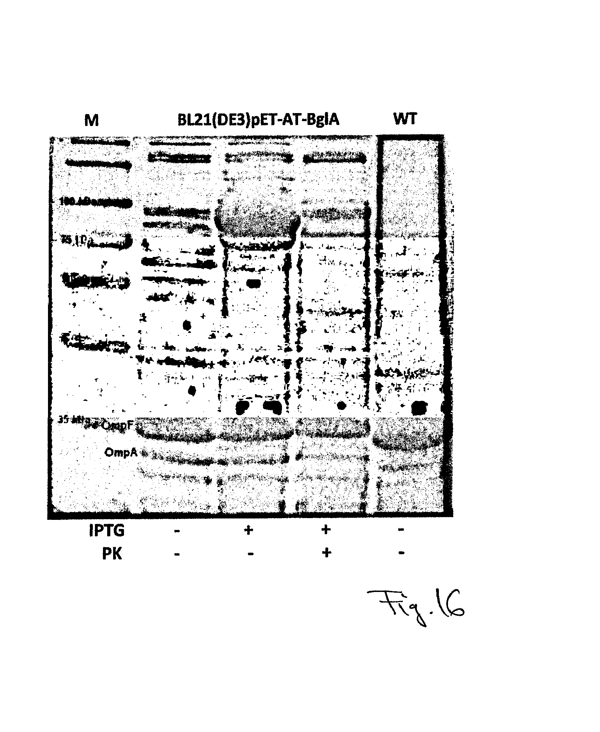

**Figur 16****:** Nachweis der Oberflächenständigkeit des BglA-Fusionsproteins auf *E. coli* BL21(DE3). Die Außenmembranproteine wurden mittels differentieller Zellfraktionierung isoliert, durch SDS-PAGE (10% Acrylamid) aufgetrennt und mit Coomassie Brilliant Blue gefärbt. M: Proteingrößenstandard; WT: Wirtsstamm; PK: Proteinase K. Der Verdau ganzer Zellen mit PK fand vor der Außenmembranprotein-Isolierung statt.

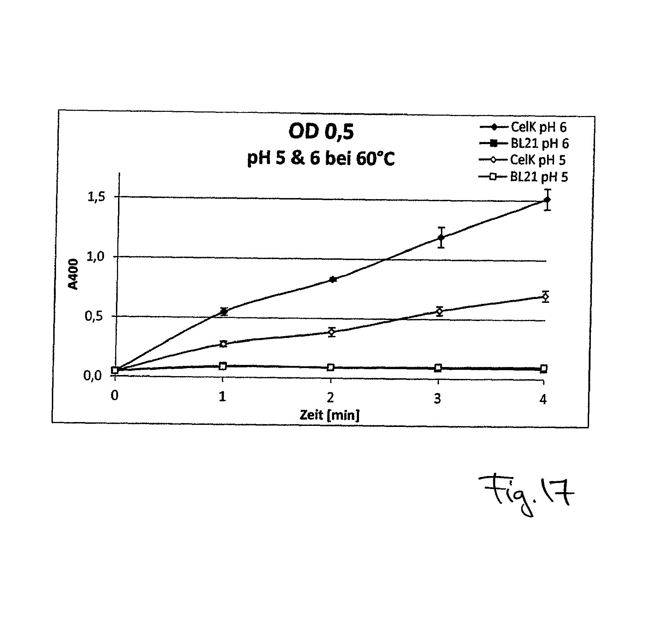

**Figur 17****:** CelK-Aktivität bei pH 5 und pH 6.Die CelK-Aktivität wurde bei 60°C und pH 5 sowie pH 6 über die Zunahme des p-Nitrophenolats bei 400 nm bestimmt. Als Substrat diente pNitrophenol-Cellobiosid und als Kontrolle der Wirtsstamm *E. coli* BL21. Abgebildet sind MW ± SD; n = 3.

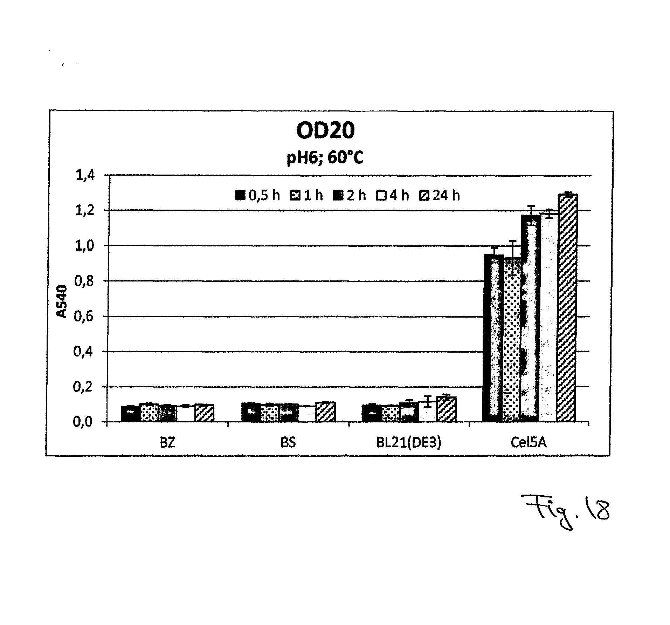

**Figur 18****:** CMCase-Aktivität des Cel5A-Stamms. Der Cel5A-Stamm und der Wirtsstamm *E. coli* BL21(DE3) wurden mit 1 % CMC versetzt und bei 60°C und pH 6 inkubiert. Nach unterschiedlichen inkubationszeiten wurden die gebildeten reduzierenden Zucker mittels DNS-Assay bei 540 nm detektiert. Blank-Zellen (BZ): nur Zellen (ohne CMC); Blank-Substrat (BS); nur CMC + Puffer (ohne Zellen). Abgebildet sind MW ± SD; n = 3.

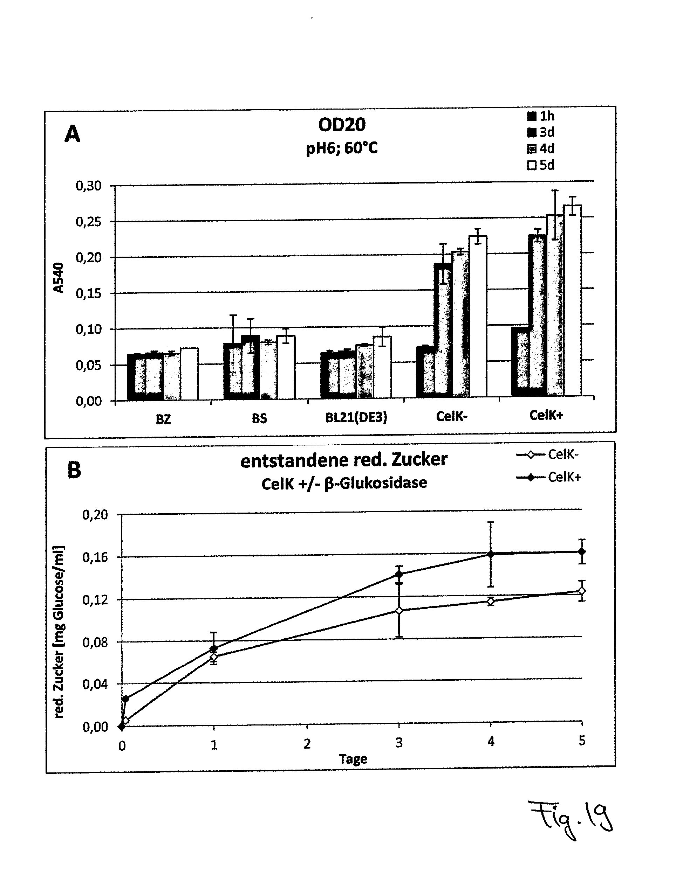

**Figur 19****:** Avicelase-Aktivität des CelK-Stamms in An-und Abwesenheit einer beta-Glucosidase. Der CelK-Stamm wurde in An-u Abwesenheit einer beta-Glucosidase (+/-) aus Mandel mit 1 % Avicel versetzt und bei 60°C und pH 6 inkubiert. Nach unterschiedlichen Zeitpunkten wurden die gebildeten reduzierenden Zucker mittels DNS-Assay bei 540 nm detektiert. Als Kontrolle diente der Wirtsstamm *E. coli* BL21. **A:** Absorptionswerte bei 540 nm. Blank-Zellen (BZ); nur Zellen (ohne Avicel); Blank-Substrat (BS): nur Avicel + Puffer (ohne Zellen), **B:** Durch den CelK-Stamm gebildete red. Zucker in mg Glucose/ml. Abgebildet sind MW ± SD; n = 3.

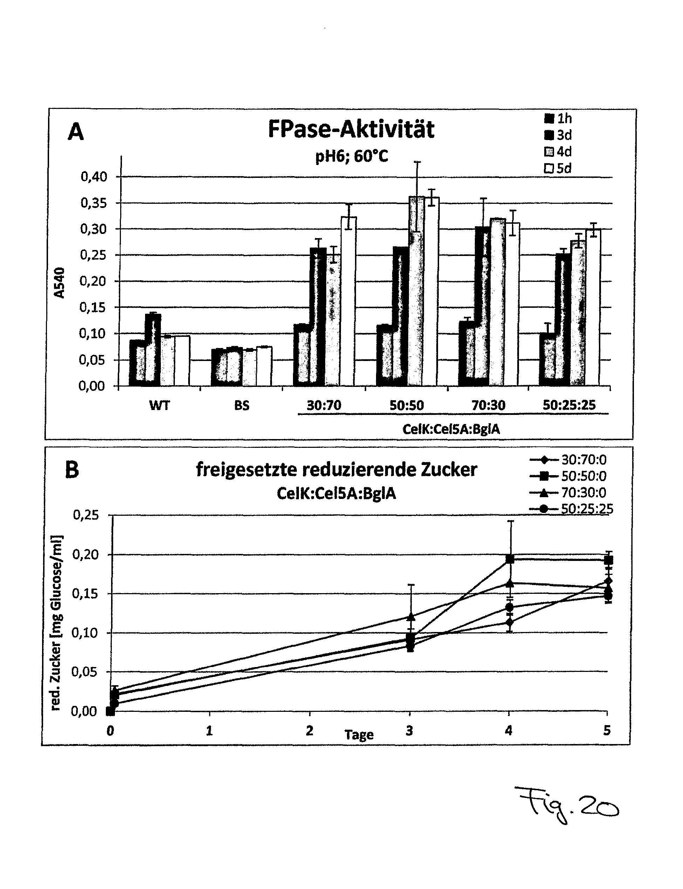

Figur 20: Filter Paper (FP) - Aktivität der Autodisplay-Cellulasen im Mikrotiterplatten-Maßstab. Die Cellulasen wurden in verschiedenen Verhältnissen mit einem FP-Plättchen (2,4 mg) bei 60°C inkubiert. Ansätze ohne BglA enthielten 0,5 U/ml beta-Glucosidase aus Mandel. Nach unterschiedlichen Inkubationszeiten wurden die gebildeten reduzierenden Zucker mittels DNS-Assay bei 540 nm detektiert **A:** Absorptionswerte bei 540 nm. WT: Wirtsstamm; Blank-Substrat (BS): FP + Puffer (ohne Zellen). B: Durch die Cellulasen gebildete red, Zucker in mg Glucose/ml. Abgebildet sind MW ± SD; n = 2.

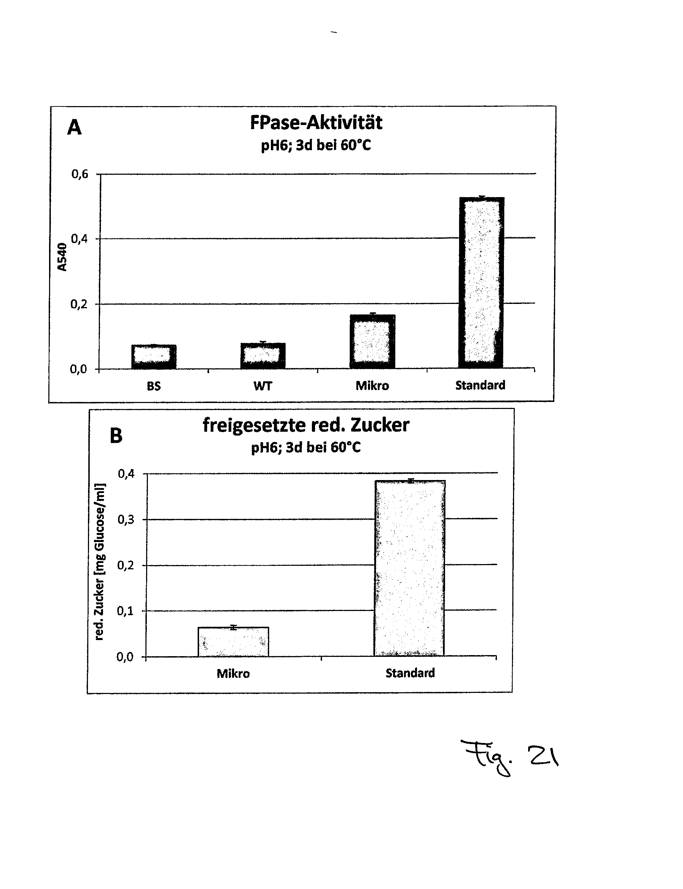

**Figur 21****:** Filter Paper (FP) - Aktivität der Autodisplay-Cellulasen: Mikrotiterplatten-Maßstab vs. Standard. Die Cellulasen wurden zu gleichen Tellen vermischt und entweder mit einem 2,4 mg Filterpapier-Plättchen (Mikro) oder mit einem 50 mg Filterpapier-Streifen (Standard) für 3 Tage bei 60°C inkubiert. Die gebildeten reduzierenden Zucker wurden mittels DNS-Assay bei 540 nm **detektiert.** A: Absorptionswerte bei 540 nm, Vor: Wirtsstamm; Blank-Substrat (BS): FP + Puffer (ohne Zellen), **B:** Durch die Cellulasen gebildete red. Zucker in mg Glucose/ml. Abgebildet sind MW ± SD; n = 2,

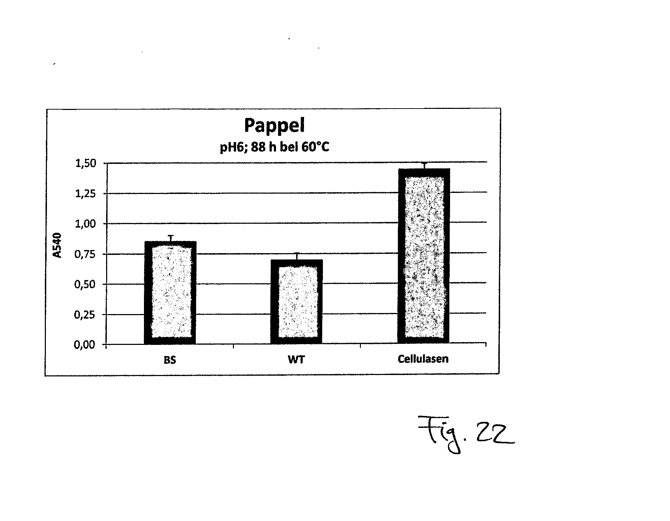

**Figur 22****:** Pappel-Abbau durch Autodisplay-Cellulasen. Die Cellulasen wurden zu gleichen Teilen vermischt und mit 4 % Pappel für 88 h bei 60°C inkubiert. Die gebildeten reduzierenden Zucker wurden mittels DNS-Assay bei 540 nm detektiert. Abgebildet sind MW ± SD; n = 2.

### Beispiele

### Allgemeines

Die Cellulasen wurden als sog. "Passagiere" in die Autodisplay-Kassette ligiert, sodass das Protein als Fusionsprotein exprimiert wird. Figur 1 zeigt den schematischen Aufbau eines solchen Konstruktes. Die Autodisplay-Kassette liegt in verschiedenen Expressionssystemen vor, sodass für die Klonierung der Cellulase-Passagiere zum einen zwei induzierbare Autodisplay-Systeme - IPTG (pET-Derivat) und Arabinose (pBAD-Derivat) - und ein konstitutives (pJM-Derivat) genutzt wurden.

Die Gensequenzen der drei Cellulasen wurden an die "codon-usage" von *E*. *coli* angepasst und von Life Technologies (GeneArt® Gene Synthesis) synthetisiert. Die optimierten Gensequenzen, sowie die daraus abgeleiteten Aminosäuresequenzen befinden sich im Sequenzprotokoll. Die Gene wurden mit Hilfe der angehängten Xho I und Kpn I Schnittstellen in die Autodisplay-Vektoren eingebracht. Für jede Cellulase wurde sowohl ein induzierbares als auch ein konstitutives Autodisplay-System verwendet. Im Falle des induzierbaren Systems wurde für die Endocellulase Cel5A und die ß-Glucosidase BglA der pET-Autodisplay-Vektor verwendet. Da CelK mit 2,4 kb sehr groß ist, wurde für CelK der pBAD-Autodisplay-Vektor eingesetzt, da dieser kleiner ist verglichen mit dem pET-Autodisplay-Vektor. Die generierten Autodisplay-cellulase-Plasmide sind somit alle < 10 kb und in Tabelle 1 zusammengefasst. Die jewelligen Plasmidkarten sind in den Figuren 2 bis 4 wiedergegeben.

**Tabelle 1:**

| Cellulase | Gen (bp) | Plasmid | Fp (kDa) | E. coli Stamm |
|---|---|---|---|---|
| Cel5A | 1422 | pET-AT-Cel5A | 102 | *E. coli* BL21(DE3)pET-AT-celSA |
| | | pJM-AT-Cel5A | | *E*. *coli* BL21pJM-AT-Cel5A |
| CelK | 2382 | pBAD-AT-CelK | 139 | *E. coli* BL21pBAD-AT-CelK |
| | | pJM-AT-CelK | | *E. coli* BL2lpJM-AT-CelK |
| BglA | 1353 | pET-AT-BglA | 101 | *E. coli* BL21(DE3)pET-AT-BglA |
| | | pJM-AT-BglA | | *E. coli* BL21pJM-AT-BglA |

Die durch IPTG induzierbaren Plasmide (pET-Derivate) wurden in *E*. *coli* BL21(DE3) (B, F-, *dcm, ompT, Ion, hsdS (rB*- *mB-) gal,* λ (DE3)) transformiert. Für die durch Arabinose induzierbaren (pBAD-Derivate) und konstitutiven Plasmide (pJM-Derivate) wurde *E. coli* BL21 (B, F-, *dcm, ompT, Ion*, *hsdS (rB- mB-) gal* ) verwendet. Die erzeugten *E. coli* Stämme sind ebenfalls in Tabelle 1 aufgelistet.

### Expressionsbedingungen der Cellulasen

Die Expression der Cellulase-Fusionsproteine wurde entweder durch Zugabe von IPTG oder mit L-Arabinose induziert. Im Falle der konstitutiven Expression ist keine Zugabe eines Induktors notwendig. Die Expressionsbedingungen der generierten Cellulase-Stämme (vgl, Tabelle 1) sind in Tabelle 2 aufgeführt. Die Zugabe von IPTG bzw. L-Arabinose erfolgte, sobald die Hauptkultur eine OD₅₇₈ von 0,5 - 0,6 erreicht hat.

**Tabelle 2:**

| Stamm | Expression des Cellulase-Fusionsproteins |
|---|---|
| *E. coli* BL21(DE3)pET-AT-Derivate | Induktion mit 1 mM IPGT für 1h bei 30 °C |
| *E. coli* BL21pBAD-AT-Derivate | Induktion mit 0,2 % L-Arabinose für 4 h bei 37 °C |
| *E. coli* BL21pJM-AT-Derivate | 24 h Kultivierung bei 37 °C (konstitutive Expression) |

Um einen eventuellen Einfluss von periplasmatisch ausgebildeten Disulfidbrücken auf den Cellulase-Transport auszuschließen, wurden die Zellen mit 10 mM ß-Mercaptoethanol (ME) kultiviert. Als Vergleich dienten Zellen, die ohne ME kultiviert wurden.

### Beispiel 1: Endocellulase Cel5A

Die Cel5A-Stämme *(E*. *coli* BL21(DE3)pET-AT-Cel5A und *E. coli* BL21pJM-AT-Cel5A) wurden gemäß Tabelle 2 ohne und mit ME angezogen und entweder für einen Aktivitätstest oder für eine Isolierung der Außenmembranproteine vorbereitet.

### Cel5A - Aktivität: induzierbar vs. konstitutiv

Die Aktivität der Endocellulase wurde mit Hilfe des CMC-Platten Assays nachgewiesen. Dabei werden Agar-Platten, bestehend aus Assay-Puffer und Substrat (Carboxymethylcellulose), hergestellt. Auf die Platten werden jeweils 10 µl Zellen (OD₅₇₈20) aufgetropft und anschließend bei 50°C Inkubiert. Die Färbung der CMC-Platten erfolgt mit Kongorot, welches Polysaccharidketten mit n > 6 Monomeren anfärbt. Abschließend werden die Platten mittels NaCl entfärbt. Wird CMC abgebaut bildet sich ein heller Hof um die Zellen, während richt hvdrolysierte CMC-Moleküle weiterhin für eine dunkelrote Färbung sorgen.

Um die optimale Inkubationszeit bei 50°C zu bestimmen, wurde eine Zeitreihe von 0,5 - 2 h ausgewählt. Neben den Cellulase-Stämmen wurde auch der Wirt auf CMC-Abbau getestet. Die Ergebnisse Ileßen erkennen, dass der Induzierbare Cel5A-Stamm eine zeitabhängige CMC-Degradation verursacht (die Höfe werden mit zunehmender Inkubationszeit größer). Im Gegensatz dazu konnte mit dem konstitutiven Cel5A-Stamm kein CMC-Abbau nachgewiesen werden. Der Wirt zeigte erwartungsgemäß keinen CMC-Abbau, Die Zugabe von ME während der Anzucht hatte keinen Einfluss,

Das Experiment hat gezeigt, dass ein CMC-Abbau mit dem Induzierbaren Cel5A-Stamm möglich ist, Die Anzucht der Zellen kann ohne Zugabe von Mercaptoethanol erfolgen. Eine Inkubationszeit der CMC-Platten von 30 min ist ausreichend.

### Cel5A-Aktivität: Nonsense Kontrolle

Um einen Einfluss des ß-barrels auf die Cel5A-Aktivität auszuschließen, wurde der CMC-Platten Assay auch mit Zellen durchgeführt, die einen anderen Passagier auf der Oberfläche tragen, welcher nicht in der Lage ist, CMC abzubauen. Dieser Stamm wird im Folgenden als Nonsense Kontrolle bezeichnet. Weiterhin wurde getestet, ob der Promotor "leaky" ist, Das bedeutet, dass das Cel5A-Fusionsproteins auch ohne Zugabe des Induktors exprimiert werden würde. Zu diesem Zweck wurde der Cel5A-Stamm nicht induziert und die Aktivität mit induzierten Zellen verglichen. Der Test ließ erkennen, dass der nicht induzierte Cel5A-Stamm einen geringen CMC-Abbau aufweist. Dies zeigt, dass ohne Induktor eine gewisse Expression des Cel5A-Fusionsproteins stattfindet. Der CMC-Abbau des induzierten Cel5A-Stamms ist jedoch stärker ausgeprägt Die Nonsense-Kontrolle zeigt genau wie der Wirtsstamm ohne Plasmid keinen CMC-Abbau. Der sehr schwach ausgebildete Hof bei Nonsense-Kontrolle und Wirt ist vergleichbar mit der Puffer-Probe. Es handelt sich somit um eine unspezifische Reaktion des Puffers auf der Agar-Platte.

Um zu gewährleisten, dass die Anzahl der ß-barred Moleküle des Cel5A-Stamms und der Nonsense Kontrolle vergleichbar ist, wurden von den getesteten Zellen noch die Proteine der Außenmembran isoliert und mittels SDS-PAGE aufgetrennt. Die *E. coli* Außenmembranproteine OmpF (37 kDa) und OmpA (35 kDa) dienen dabei als Markerproteine für die Außenmembranfraktion. Das Ergebnis ist in Figur 5 dargesteilt. Nach der Induktion mit IPTG konnten vergleichbar starke Banden für das Cel5A-Fusionsprotein und das Fusionsprotein der Nonsense-Kontrolle nachgewiesen werden, welche gut mit den errechneten MW von ca. 102 kDa bzw. 96 kDa übereinstimmen. Bei dem nicht induzierten Cel5A-Stamm konnte keine Bande für das Cel5A-Fusionsprotein nachgewiesen werden. Ebenso zeigte der Wirt erwartungsgemäß keine Bande für das Cel5A-Fusionsprotein und die Nonsense-Kontrolle.

Es konnte demnach gezeigt werden, dass es sich bei dem CMC-Abbau durch den Cel5A-Stamm um eine spezifische Cellulase-Reaktion handelt.

### Lagerstabilität des Cel5A-Stamms

Um zu testen, ob der Cel5A-Stamm bei 4°C gelagert werden kann, wurde die Aktivität der Zellen aus Versuch 3.2 nach 10 Tagen Lagerung bestimmt. Aus dem Test wurde ersichtlich, dass der gelagerte Cel5A-Stamm keinen Aktivitätsvedust fn diesem Zeitraum aufweist. Der CMC-Abbau ist mit frischen Zellen vergleichbar. Der Wirt und die Nonsense-Kontrolle zeigten keinen CMC-Abbau.

Der Versuch zeigte dass es möglich ist, den Cel5A-Stamm für mindestens 10 Tage bei 4°C ohne Aktivitätsverlust zu lagern.

### Oherflächenständigkeit des Cel5A-Stamms

Die erfolgreiche Expression des Cel5A-Fusionsproteins konnte im Vorstehenden gezeigt werden. Der Nachweis des Fusionsproteins in der Außenmembran bietet jedoch noch keinen Aufschluss über deren Orientierung. Die Enzyme können, wie erwünscht zum Extrazellularraum oder aber zum Periplasma hin orientiert vorliegen. Durch die Inkubation der intakten Bakterienzellen mit Proteinase K und anschließender Präparation der bakteriellen Außenmembranproteine ist es möglich, die Oberflächenständigkeit eines Autodisplay-Passagiers nachzuweisen. Da Proteinase K - Moleküle aufgrund ihrer Größe nicht in der Lage sind, die äußere Membran von *E*. *coli* zu überwinden, resultiert die Inkubation ganzer Zellen mit diesen Proteasen lediglich im Verdau von Proteinem die sich an der Oberfläche der Außenmembran befinden. Die Außenmembranproteine OmpF und OmpA sind vor einem Proteaseverdau geschützt, da es sich hierbel um Integrale Membranproteine handelt. Ihr Nachweis nach Protease-Behandlung kann daher als Indiz für die Unversehrtheit der Zeilen angesehen werden.

Um die Obeflächenständigkeit nachzuweisen wurde der Cel5A-Stamm nach der Induktion der Cel5A-Expression mit Proteinase K inkubiert. Als Vergleich dienten induzierte Zellen, die nicht mit der Protease behandelt werden, Der *E. coli* Wirtsstamm sowie der nicht induzierte Cel5A-Stamm wurden als Kontrollen eingesetzt. In Figur 6 ist die bereits gezeigte starke Expression des Cel5A-Fusionsproteins nach IPTG-Induktion bei ca. 102 kDa zu erkennen (vgl. Abbildung 4). Erwartungsgemäß konnte weder im *E. coli* Wirtsstamm noch im nicht induzierten Cel5A-Stamm eine Expression bei ca. 102 kDa nachgewiesen werden. Aufgrund der starken Cel5A-Epression ist ein vollständiger proteolytischer Abbau des Cel5A-Fusionsproteins schwierig. Die Abnahme der Cel5A-Bande bei gleichzeitigem Erhalt der OmpF- und OmpA- Banden nach Protease-Behandlung spricht eindeutig für eine Orientierung der Cel5A nach außen.

### Beispiel 2: Exocellulase CelK

Die CelK-Stämme *(E*. *coli* BL21pBAD-AT-CelK und *E*. *coli* BL21pJM-AT-Cel5K) wurden gemäß Tabelle 2 ohne und mit Mercaptoethanol kultivlert und entweder für einen Aktivitätstest oder für eine Isollerung der Außenmembranproteine vorbereitet.

### CelK Aktivität: p-Nitrophenolat-Assay

Die Aktivität der Exocellulase wurde photometrisch über die Zunahme von p-Nitrophenolat bestimmt. Als Substrat diente dabei p-Nitrophenol-Cellobiosid. In der Literatur wird ein Absorptionsmaximum von 380 - 420 nm für das p-Nitrophenolat beschrieben. Um die optimale Wellenlänge für die Messung von p-Nitrophenolat zu ermitteln, wurde zuerst ein Spektrum unter celK-Assay-Bedingungen aufgenommen, Dazu wurde p-Nitrophenol in Natdum-Citrat-Puffer pH 6 angesetzt und bei 60°C inkubiert. Da die Gelbfärbung des p-Nitrophenolats am stärksten im Alkalischen ausgeprägt ist, wurde die Probe noch mit Natriumcarbonat alkalisiert und dann in der Mikrotiterplatte photometrisch vermessen. Figur 7 zeigt, dass unter CelK-Bedingungen eine p-Nitrophenalat-Messung bei ca. 360-450 nm möglich ist. Angelehnt an die Literatur wurde eine Absorptionsmessung bei 400 nm für den nachfolgenden CelK-Assay festgelegt.

Um die Aktivität der CelK-Stämme zu bestimmen, wurde eine Standardkurve mit p-Nitrophenolat angefertigt (0 mM - 1 mM). Dabei wurden die p-Nitrophenolat-Proben entsprechend den CelK-Proben behandelt und unverdünnt bzw. 1:2 verdünnt vermessen

### CelK - Aktivität; konstitutiv versus induzierbar

CelK-Vorversuche hatten gezeigt, dass der induzierbare CelK-Stamm, verglichen mit dem konstitutiven CelK-Stamm, eine höhere Aktivität aufweist. Um einen genaues Verhältnis zu bestimmen, wurden die Aktivitäten im direkten Vergleich ermittelt. Das Ergebnis ist in Figur 8 dargesteilt. Für den induzlerbaren CelK-Stamm konnte anhand einer Standard-Kurve eine Aktivität von 210 mU/OD₅₇₈1/ml ermittelt werden. Für den konstitutiven CelK-Stamm konnte bei der eingesetzten OD₅₇₈1 nach 3 min bei 60°C kein Umsatz beobachtet werden. Daher wurde der konstitutive CeiK-Stamm (OD₅₂₈1) für 30 min bei 60°C inkubiert. Nun konnte eine Absorption bei 400 nm von 0,25 bestimmt werden, was einer Aktivität von 2,2 mU/OD₅₇₈1/ml entspricht. Der induzierbare CelK-Stamm ist damit ca. 100-fach aktiver verglichen mit dem konstitutiven celK-Stamm. Die weiteren Versuche wurden daher mit dem induzierbaren CelK-Stamm (Kultivierung ohne Mercaptoethanol) fortgesetzt.

### CelK-Aktivität: Nonsense-Kontrolle

Entsprechend der Endocellulase solite auch für die Exocellulase eine Nonsense-Kontrolle auf Aktivität getestet werden. Aus Abbildung 12 wurde ersichtlich, dass die OD₅₇₈ des CelK-Stamms im Ansatz: ≤ 1 sein soil. In dem folgenden Versuch wurde OD₅₇₈0,5 ausgetestet. Da die p-Nitrophenolat-Bildung nach ca. 3 min schon sehr ausgeprägt war (vgl. Figur 8), wurde in diesem Versuch eine Zeitreihe von 0 - 4 min ausgewählt. Dazu wurden der CelK-Stamm, die Nonsense-Kontrolle sowie der Wirtsstamm mit p-Nitrophenol-Celloblosid versetzt und nach jeder Minute das gebildete p-Nitrophenolat bei 400 nm gemessen. Die Proben wurden vor der Alkalisierung 1:2 verdünnt Das Ergebnis ist in Figur 9 dargesteilt Es hat sich gezeigt das OD₅₇₈0,5 ausreichend für die Aktivitäts-Messung ist. Für den CelK-Stamm konnte anhand der Standard-Kurve (1:2 verdünnt) eine Aktivität von ca. 300 mU/OD₅₇₈1/ml errechnet werden. Betrachtet wurden dabei die ersten 3 min. Danach ist die Reaktion nicht mehr linear. Die Nonsense-Kontrolle zeigte wie der Wirtsstamm keinen Umsatz des Substrates.

Um zu überprüfen, ob die Expression des CelK-Fusionsproteins und des Fusionsproteins der Nonsense-Kontrolle gleich ist, wurden neben dem Aktivitätstest noch die Proteine der Außenmembran isoliert und mittels SDS-PAGE aufgetrennt. Die *E. coli* Außenmembran-proteine OmpF (37 kDa) und OmpA (35 kDa) dienen dabei: als Markerproteine für die Außenmembranfraktion. Das Ergebnis ist in Figur 10 dargesteilt, Nach der Induktion mit Arabinose konnten vergleichbar starke Banden für das CelK-Fusionsprotein und das Fusionsprotein der Nonsense-Kontrolle nachgewiesen werden, weiche gut mit den errechneten MW von ca. 139 kDa bzw. 103 kDa übereinstimmen. Bel dem Wirtsstamm konnte keine Bande der beiden Fusionsproteine beobachtet werden.

Mit diesem Versuch konnte gezeigt werden, dass es sich bei der Umsetzung des p-Nitrophenolats um eine spezifische celK-Reaktion handelt.

### Lagerstabilität des CelK-Stamms

Um zu testen, ob der CelK-Stamm bei 4°C gelagert werden kann, wurde die Aktivltät der Zellen aus dem vorstehenden Versuch nach 8 Tagen Lagerung bestimmt. Aus Figur 11 wird ersichtlich, dass der gelagerte CelK-Stamm keinen Aktivitätsverlust in diesem Zeitraum aufweist. Die Bildung von p-Nitrophenolat ist mit frischen Zellen vergleichbar (Figur 9). Auch hier konnte eine Aktivität von ca. 300 mU/OD₅₇₈1/ml erzielt werden. Der Wirt und die Nonsense-Kontrolle zeigten keinen Umsatz des Substrats.

Der Versuch zeigte, dass es möglich ist, den CelK-Siamm für mindestens 8 Tage bei 4°C ohne Aktivitätsverlust zu lagern.

### Oberfächenständigkeit des CelK-Stamms

Die erfolgreiche Expression des CelK-Fusionsproteins konnte in Figur 10 bereits gezeigt werden. Um die Orientierung des Enzyms nach außen zu bestätigen wurde der CelK-Stamm mit Proteinase K behandeit und anschließend die Außenmembranproteine isoliert. OmpF und OmpA wurden als Nachweis für die Unversehrtheit der Zeilen nach Protease-Behandiung herangezogen.

Der CelK-Stamm wurde nach der Induktion der CelK-Expression mit Proteinase K inkubiert. Als Vergleich dienten induzierte Zellen, die nicht mit der Protease behandeit wurden. Der *E. coli* Wirtsstamm sowie der nicht induzierte CelK-Stamm wurden als Kontrolien eingesetzt. In Figur 12 ist die bereits gezeigte starke Expression des Celk-Fusionsproteins nach Arabinose-Induktion bei ca. 139 kDa zu erkennen. Erwartungsgemäß konnte weder im *E. coli* Wirtsstamm noch im nicht induzierten CelK-Stamm eine Expression bei ca. 139 kDa nachgewiesen werden. Aufgrund der starken CelK-Expression ist ein vollständiger proteolytischer Abbau des CelK-Fusionsproteins schwierig. Die CelK-Bande ist 2war vollständig verschwunden, aber die OmpA-Bande ist im Vergleich zum Wirtsstamm auch etwas schwächer. Nichtsdestotrotz spricht dies eindeutig für eine Orientierung des Enzyms nach außen,

### Beispiel 3: beta-Glucosidase BglA

### BglA-Expression und -Aktivität: induzierbar vs. konstitutiv

Die Expression des Fusionsproteins erfolgte im Faile des konstitutiven Systems für 24 h bei 37°C. Die Induktion der Expression im pET-System wurde mittels einstündiger Inkubation der Zellen mit 1 mM IPTG bei 30°C erzielt, nachdem sie eine OD₅₇₈ von 0,6 erreicht hatten. Eine Zugabe von 10 mM ß-Mercaptoethanol während der Kultvierung ist nicht nötig, da die BglA-Aminosäuresequenz nur ein Cystein enthält und somit keine Disulfidbrücken im Periplasma ausgebildet werden können, die den Transport beeinflussen konnten.

Nachdem die bei den BglA-Stämme (induziert und konstitutiv) generiert wurden, solite die Expression des BglA-Fusionsproteins in der Außenmembran von *E. coli* überprüft werden. Dazu wurden die Proteine der Außenmembran beider Stämme isoliert und mittels SDS-PAGE aufgetrennt. Die *E*. *coli* Außenmembranproteine OmpF (37 kDa) und OmpA (35 kDa) dienen dabei als Markerproteine für die Außenmembranfraktion. Das Ergebnis ist in Figur 13 dargestellt. Im Falle des induzierbaren Expressionssystems konnte nach der Induktion mit IPTG eine starke Bande für das BglA-Fusionsprotein nachgewiesen werden, welche gut mit dem errechneten MW von ca. 101 kDa übereinstimmt. Für den nicht induzierten BglA-Stamm sowie für den Wirtsstamm konnte erwartungsgemäß keine Bande für das BglA-Fusionsprotein nachgewiesen werden. Im konstitutiven System konnte keine Expression des BglA-Fusionsproteins nachgewiesen werden. Erfahrungen haben gezeigt, dass der Nachwels eines konstitutiv exprimierten Passagiers mittels Coomassie-Färbung nicht sensitiv genug ist.

Nachdem die Expression des BglA-Fusionsproteins überprüft wurde, wurde die BglA-Aktivität des konstitutiven und induzierbaren Stamms mittels pNitrophenol-Glucopyranosid als Substrat bestimmt. Die Aktivität des BglA-Stamms wurde photometrisch über die Bildung von p-Nitrophenolat bei 400 nm bestimmt. Im Gegensatz zur Exocellulase CelK diente dabei p-Nitrophenol-Glucopyranosid als Substrat. Um den Assay zu etablieren, wurde der Test zuerst mit einer gekauften beta-Glucosidase (aus Mandel) durchgeführt Dazu wurden die optimalen Bedingungen für dieses Enzym gewählt (pH 5 und 35°C). Eingesetzt wurden zwei verschiedenen Konzentrationen. Es war eine konzentrationsabhängige Aktivität der beta-Gluco-sidase zu erkennen. Der Blank zeigt erwartungsgemäß keine p-Nitrophenolat Zunahme.

Die BglA-Aktivität wird über die Absorptionsmessung des entstehenden pNitrophenolat bei 400 nm bestimmt (Gelbfärbung). Die Reaktion erfolgte, angelehnt an die publizierten, optimalen Bedingungen für die BglA bei einem pH-Wert von 6,0 und einer Inkubationstemperatur von 60°C. Da die Inkubationsdauer des BglA-Stamms mit dem Substrat nicht bekannt war, wurden die Reaktionsansätze solange inkubiert, bis eine deutliche Gelbfärbung (pNitrophenolat) zu erkennen war. Diese war im Falle des induzierbaren BglA-Stamms nach 1 h bei 60°C zu verzeichnen. Vor der photometrischen Messung wurden die Zellen abgetrennt und der Überstand in eine 96 well-Mikrotiterplatte überführt. Nach der Alkalisierung durch Zugabe von Natriumcarbonat erfolgte die Absorptionsmessung bei 400 nm. In Figur 14 ist zu erkennen, dass der induzierbare BglA-Stamm in der Lage war, dass pNitrophenol-Glucopyranosid umzusetzen (pNitrophenolat-Zunahme bei 400 nm). Im Gegensatz dazu zeigte der konstitutive BglA-Stamm keine Entstehung von p-Nitrophenolat innerhalb 1 h bei 60°C. Eine längere Inkubationszeit wurde nicht ausgetastet. Sowohl der Blank als auch der Wirtsstamm zeigten keine Umsetzung des Substrats. Wie bereits bei den anderen beiden Cellulasen konnte mit dem induzierbaren Expressionssystem eine bessere Aktivität erreicht werden.

### BglA-Aktivität: Nonsense-Kontrolle

Nachdem gezeigt werden konnte, dass der induzierbare BglA-Stamm aktiv ist, sollte noch eine Zeitrelhe aufgenommen werden, um die Aktivität besser bestimmen zu können. Gleichzeitig wurde auch, entsprechend den anderen beiden cellulasen, eine sogenannte Nonsense-Kontrolle getestet. Dabei handelt sich um einen *E*. *coli-Stamm,* der einen anderen Passagier an der Oberfläche präsentiert, der nicht in der Lage ist, das Substrat umzusetzen. Ausgewählt wurde der CelK-Stamm. Es handelt sich zwar um eine Cellulase, aber CelK kann pNitrophenol-Glucopyranosid nicht umsetzen. Um die BglA-Aktivität bestimmen zu können, wurde zuerst eine pNitrophenol-Standardkurve aufgenommen. Anschließend wurden der BglA-Stamm, die Nonsense-Kontrolle (CelK-Stamm) sowie der Wirtsstamm mit p-Nitrophenol-Glucopyranosid versetzt und nach verschiedenen Inkubationszeiten bei 60°C das gebildete p-Nitrophenolat bei 400 nm gemessen. In Figur 15 ist zu erkennen, dass der BglA-Stamm eine kontinulerliche pNitrophenolat-Zunahme über die Zeit aufweist. Anhand der Standardkurve konnte eine BglA-Aktivität von ca. 1 mU/ml/OD1 errechnet werden. Die Nonsense-Kontrolle zeigte wie der Wirtsstamm *E*. *coli* BL21(DE3) keinen Umsatz des Substrates.

### Oberflächenständigkeit der BglA auf E. coli BL21(DE3)

Die erfolgreiche BglA-Aktivität des induzierbaren Stamms konnte in Figur 14 gezeigt werden, die Expression des BglA-Fusionsproteins ist in Figur 13 dargestellt. Um die Oberflächenständigkeit nachzuweisen, wurde der BglA-Stamm nach der Induktion der BglA-Expression mit Proteinase K inkubiert. Die Protease kann aufgrund ihrer Größe nicht in die *E*. *coli* zelle gelangen und baut daher nur Proteine ab, die sich an der Oberfläche der Zelle befinden. Als Vergleich dienten induzierte Zellen, die nicht mit der Protease behandelt wurden. Der *E. coli* Wirtsstamm sowie der nicht induzierte BglA-Stamm wurden als Kontrollen eingesetzt. Bei OmpA und OmpF handelt es sich um integrale Membranproteine die vor einem Proteaseverdau geschützt sind. Diese dienen daher als Indiz für die Unversehrtheit der Zelle. In Figur 13 ist die bereits gezelgte starke Expression des BglA-Fusionsproteins nach IPTG-Induktion bei ca. 101 kDa zu erkennen. ErwartungsgemäB konnte weder im *E. coli* Wirtsstamm noch im nicht induzierten BglA-Stamm eine Expression bei ca. 101 kDa nachgewiesen werden (Figur 16). Aufgrund der starken BglA-Expression ist ein vollständiger proteolytischer Abbau des BglA-Fusionsproteins schwierig. Die Abnahme der BglA-Bande bei gleichzeitigem Erhalt der OmpF- und OmpA- Banden nach Protease-Behandlung spricht nach unserer Erfahrung eindeutig für eine Orientierung der BglA nach außen.

### Beispiel 4

Mit Hilfe der Autodisplay-Technologie war es möglich, drei aktive Cellulasen auf der Oberfläche von *E. coli* zu präsentieren. Dabei war ein induzierbares Expressionssystem in allen Fällen das Beste. Die Cellulase-Stämme und ihre Kultivierung sind in Tabelle 2 zusammengefasst.

Um die Autodisplay-Cellulasen zusammen für einen synergistischen Cellulose-Abbau einsetzen zu können, müssen gemeinsame Parameter wie pH-Wert und Temperatur festgelegt werden. Exocellulase und B-Glucosidase weisen ein pH-Optimum von 6,0 auf. Das Temperaturoptimum liegt bei 60°C. Die Endocellulase besitzt jedoch ein pH-Optimum von 5,0 und ein Temperaturoptimum von 50°C.

### Ermittlung der gemeinsamen Temperatur

Da zwei der drei Cellulasen (CelK und BglA) ein Temperaturoptimum bei 60°C aufweisen, sollte getestet werden, ob die Endocellulase Cel5A ebenfalls bei 60°C aktiv ist. In der Literatur ist eine geringe Abnahme der Cel5A-Aktivität (ca. 5-10 %) bei 60°C, verglichen mit der Aktivität bei 50°C beschrieben (Lin L et al, 2009). Die Aktivität der Cel5A wurde mit Hllfe des CMC-Platten Tests bei pH 5bestimmt. Zu diesem Zweck wurden Cel5A-Zellen auf eine CMC-Platte getropft und 30 min bei 50°C, 55°C und 60°C inkubiert. Als Kontrolle diente der Wirtsstamm *E*. *coli* BL21(DE3) und Puffer. Die Färbung der CMC-Platten erfolgte mit Kongorot. Abschließend wurden die Platten mit NaCl entfärbt. Es konnte festgestellt werden, dass die Cel5A-Zellen bei jeder eingesetzten Temperatur CMC gleich gut abbauen (heller Hof), wohingegen der Wirtsstamm und der Puffer negativ sind. Die Cel5A-Zellen können somit auch bei 60°C angesetzt werden. Im Folgenden wurden daher bei gleichzeitigem Einsatz aller drei Cellulasen bei 60°C gearbeitet.

### Ermittlung des gemeinsamen pH-Werts

Exocellulase (CelK) und B-Glucosidase (BglA) haben ein pH-Optimum bei pH 6. Die Endocellulase (Cel5A) weist hingegen ein pH-Optimum von 5 auf. In der Literatur ist eine Abnahme der Cel5A -Aktivität bei pH 6 gegenüber pH 5 beschrieben (Lin L et al, 2009). Daher sollte getestet werden, ob es möglich ist CelK bei pH 5 einzusetzen. Zu diesem Zweck wurde der CelK-Stamm nach Induktion der Proteinexpression zum einen in Natriumcitrat-puffer pH 6 und zum anderen in Natriumacetatpuffer pH 5 geemtet. Die CelK-Aktivität wurde anhand der Zunahme des p-Nitrophenolats bei 400 nm durch Abbau des pNitrophenol- Cellobiosids (pNPC) bei 60°C bestimmt. Als Kontrolle diente der Wirtsstamm *E*. *coli* BL21. Figur 17 zeigt, dass bei beiden eingesetzten pH-Bedingungen eine pNitrophenolat-Zunahme zu erkennen ist, während der Wirtsstamm unverändert bleibt. Weiterhin ist zu erkennen, dass bei pH 6 verglichen mit pH 5 mehr pNitrophenolat entsteht. Bei pH 6 konnte eine CelK-Aktivität von durchschnittlich 270 mU/ml/OD1 erzielt werden. Bei pH 5 hingegen nur ca. 110 mU/ml/OD1. Somit führt eine pH-Senkung im Assay von pH 6 auf pH 5 zu einer 60 %igen Abnahme der CelK-Aktivität.

Für die Endocellulase Cel5A wird ebenfalls ein Aktivitätsverlust beschrieben, wenn der pH von 5 auf 6 angehoben wird. Da jedoch auch die ß-Glucosidase BglA ein pH-Optimum bei 6 hat, werden die nachfolgenden Assays bei pH 6 durchgeführt, so dass nur ein Enzym unter nicht optimalen pH-Bedingungen eingesetzt werden muss.

### Nachweis reduziender Zucker: Etablierung des DNS-Assays

Die gemeinsame Cellulase-Aktivität der Autodisplay-Cellulasen sollte nun an Cellulose-Substraten getestet werden, Dies erfolgt u.a. colorimetrisch durch Messung der freigesetzten, reduzierenden Zucker nach der Dinitrosalicylsäure-Methode (Miller GL 1959). Die Methode verbindet die Oxidation einer Carbonylverbindung mit der Reduktion einer Nitrogruppe in 3,5-Dinitrosalicylsäure unter alkalischen Bedingungen. Dabei wird 3,5-Dinitrosalicylsäure zu 3-Amino-5-nitrosalicylsäure reduziert, welches ein Absorptionsmaximum bei 540 nm aufweist. Bei dieser Reaktion schlägt die Farbe der DNS-Lösung von orangegelb nach rot um.

### CMCase-Aktivität der Endocellulase Cel5A

Bisher wurde die Cel5A-Aktivität indirekt mittels CMC-Platten Test ermittelt. Mit Hilfe der DNS-Methode können die gebildeten, reduzierenden Enden der Carboxymethylcellulose durch die Cel5A-Reaktion quantitativ bestimmt werden. Dazu wurde der Cel5A-Stamm mit 1 % CMC versetzt und bei 60°C inkubiert. Nach unterschiedlichen Inkubationszeiten wurden die Zellen abgetrennt und der Überstand mit DNS-Reagenz versetzt. Nach der Inkubation bei 95°C und 4°C wurde die Absorption bei 540 nm photometrisch bestimmt, Als Vergleich diente der Wirtsstamm *E*. *coli* BL21(DE3). Als Kontrollen wurden nur Zellen (BZ) bzw. nur CMC (BS) eingesetzt. In Figur 18 ist zu erkennen, dass der Cel5A-Stamm reduzierende Enden bildet, die mittels DNS-Reagenz erfasst werden können. Aufgrund der Carboxymethyl-Substitutionen ist der CMC-Abbau keine lineare Reaktion, da die Endocellulase durch den Grad der Substitution beeinflusst wird. Daher soll ein Umsatz von ca. 5 % nicht überschritten werden. Nach 30 min bei 60°C setzt der Cel5A-Stamm ca. 8 % des CMC um. Um eine bessere Einschätzung der Cel5A-Aktivität zu erreichen, sollte die Inkubation bei 60°C < 30 min sein. Der Wirtsstamm sowie die beiden anderen eingesetzten Kontrollen zeigen erwartungsgemäß keine Endocellulase-Aktivität.

### Beispiel 5: Avicelase-Aktivität der Exocellulase CelK

Die Exocellulase-Aktivität wurde bisher mit pNitrophenol-Cellobiosid bestimmt. Dabei handelt sich um ein lösliches und somit einfach umzusetzendes Substrat. Um die CelK-Aktivität an kristallinen Celluloseregionen zu testen, wurde Avicel (mikrokristalline Cellulose) als Substrat genutzt. Dazu wird der CelK-Stamm mit 1 % Avicel bei 60°C inkubiert und nach unterschiedlichen Inkubationszeiten die freigesetzten reduzierenden Zucker mittels DNS-Assay bei 540 nm bestimmt. Die Konzentration der reduzierenden Zucker wird anhand einer Glucose-Standardkurve in mg Glucose/ml ermittelt. Da die Exocellulase Cellobiose freisetzt, wurde während des Avicel-Umsatzes eine β-Glucosidase zuzugeben, welche die Cellobiose in zwei reduzierende Zuckereinheiten in Form von Glucose abbaut. Dies ermöglicht eine genauere Bestimmung der Menge an gebildeten reduzierenden Zucker, da diese anhand einer Glucose-Standardkurve berechnet wird. Gleichzeitig stellt die Cellobiose einen inhibitor der Exocellulase dar, der mit Hilfe der β-Glucosidase entfernt wird. Die Umsetzung von Avicel wurde in An- und Abwesenheit einer kommerziell erhältlichen, aufgereinigten β-Glucosidase aus Mandel untersucht. Als Vergleich diente der Wirtsstamm *E*. *coli* BL21. Als Kontrollen wurden nur Zeifert (BZ) bzw. nur Substrat (BS) eingesetzt. Aus Figur 19A wird ersichdich, dass der CelK-Stamm in der Lage ist, mikrokristalline Cellulose abzubauen. Der Wirtsstamm, sowie die eingesetzten Kontrollen zeigen keine Zunahme von reduzierenden Zuckern. Die Konzentration der freigesetzten reduzierenden Zucker ist in Figur 19B dargestellt. Wie erwartet liegt bei Anwesenheit der ß-Glucosidase aus Mandel eine höhere Konzentration vor, verglichen mit dem Umsatz ohne ß-Glucosidase.

### B-Glucosidase BglA: DNPGase-Aktivität

Der kommerziell erhältliche Cellulase-Mix Celluclast ® (Novozymes) enthält Cellulasen aus dem Pilz *Trichoderma reseel* ATCC 26921. Dieser Mix katalysiert den Abbau von cellulose in Glucose, Cellobiose und höhere Glucose-Polymere. Für eine bestmögliche Cellulose-Umsetzung wird zusätzlich der Elnsatz einer B-Glucosidase aus *Aspegillus niger* (Novozym© 188) empfohlen. Als Dosierung wird von Novozyme 1 % Celluclast und 0,2 % Novozym188 beschrieben. Die Celluclast 0,2 optimalen Bedingungen für Cellulclast sind eine Temperatur von 50-60°C und ein pH-Wert von 4,5-6,0. Daher konnten zum Vergleich die Reaktionsbedingungen der Autodisplay-Cellulasen von 60 °C und pH 6 übernommen werden.

Die ß-Glucosidase Aktivität wurde mit pNitrophenol-Glucopyranosid (pNPG) über die Zunahme von pNitrophenol-Glucopyranosid bei 400 nm photometrisch (pNPGase-Aktivität) bestimmt Dazu wurde der BglA-Stamm (OD₅₇₈20) mit pNPG für 15 min bei 60°C inkubiert. Als Kontrolle diente der Wirtsstamm *E. coli* BL21(DE3) und Puffer (Blank). Im Vergleich dazu wurden 1 % Celluclast und 0,2 % Novozym188 mit pNPG bei 60°C inkubiert Da schon nach 3 min eine starke Gelbfärbung zu erkennen war, wurde der pNitrophenolatgehalt nach 3 min gemessen, statt nach 15 min, entsprechend der BglA. 1 % Celluclast sowie 0,2 % Novo188 zeigen verglichen mit dem BglA-Stamm, der in einer Konzentration von OD₅₇₈20 eingesetzt wurde, eine stärkere Zunahme des pNitrophenolats. Die Kontrollen zeigten wie erwartet keine pNPGase-Aktivität.

Mit Hilfe des pNitrophenolat-Standard wurde die B-Glucosidase-Aktivität in U/I errechnet (vgl. Tabelle 3). Novozym188 zeigte dabei eine 14fach bessere Aktivität verglichen mit dem BglA-Stamm. Celluclast wies eine 12fach stärkere Aktivität auf.

**Tabelle 3:**

| Cellulase | U/I |
|---|---|
| BglA | 22 |
| Novozymn188 | 312 |
| Celluclast | 264 |

### Exocellulase Celk: pNPCase-Aktivität

Die Aktivität der Exocellulase wurde aus praktischen Gründen mit pNitrophenol-Cellobiosid (pNPC) über die Zunahme von pNitrophenolat bei 400 nm photometrisch bestimmt (pNPCase-Aktivität), Dazu wurde der CelK-Stamm (00₅₇₈0,15) und Celluclast (1%) mit pNPC für 4 min bei 60°C ijnkubiert. Als Kontrolle diente der Wirtsstamm *E. coli* BL21 und Puffer (Blank). Der CelK-Stamm mit einer OD₅₇₈0,5 zeigte eine stärkere pNitrophenolat-Zunahme verglichen mit 1 % Celluclast. Die beiden eingesetzten Kontrollen zeigten erwartungsgemäß keine pNPCase-Aktivtät. Mit Hilfe des pNitrophenolat-Standards wurde die CelK-Aktivität in U/I errechnet (vgl. Tabelle 4). Der CelK-Stamm wies eine doppeit so hohe pNPCase-Aktivität, verglichen mit Celluclast auf.

**Tabelle 4:**

| Cellulase | U/I |
|---|---|
| Celk | 108 |
| Celluclast | 52 |

### Endocellulase Cel5A: CMCase-Aktivität

Die Endocelulase-Aktivität wurde mit CMC über die Bestimmung der red. Zucker mittels DNS-Assay ermittelt (CMCase-Aktivität). Dazu wurde der Cel5A-Stamm (OD₅₇₈20) und Celluclast 1% mit CMC für 15 min bei 60°C inkubiert. Als Kontrollen dienten der Wirtsstamm *E. coli* BL21(DE3). und Puffer (Blank). Mit Celluclast konnte eine stärkere Frelsetzung von reduzierenden Zuckern beobachtet werden, verglichen mit dem Cel5A-Stamm. Mit Hilfe der Glucose-Standardkurve wurde die CMCase-Aktivität in U/I berechnet (vgl. Tabelle 5). Celluclast zeigt eine 2,4fach bessere CMCase-Aktivität gegenüber dem Cel5A-Stamm. CMC ist jedoch nicht spezifisch für Endocellulasen. Exocellulasen und B-Glucosidasen setzen ebenfalls geringe Mengen reduzierende Zucker aus CMC frei. Da Celluclast ein Mix aus *Trichoderma* Cellulasen ist, wird CMC per se besser umgesetzt, vergehen mit dem Cel5A-Stamm, der nur eine Endocellulase auf der Oberfläche präsentiert. Die 2,4fach bessere CMCase-Aktivität des Celluclast ist daher nicht mit einer 2,4fach besseren Endocellulase-Aktivität gleichzusetzen.

**Tabelle 5:**

| Cellulase | U/I |
|---|---|
| Cel5A | 227 |
| Celluclast | 535 |

### Beispiel 6: Recycling der Autodisplay-Cellulasen

Ein Vorteil der Autodisplay-Cellulasen gegenüber löslitchen Enzymen ist die einfache Rückgewinnung des Gansell-Katalysators aus dem Reaktionsansatz. Die Zellen werden dazu sedimentiert, im geeigneten Puffersystem resuspendiert und stehen dann für einen weiteren Umsatz zur Verfügung. Um zu testen, ob der Prozess des Recyclings sich auf die Aktivität der Cellulasen auswirkt, wurden die Cellulase-Stämme nach Bestimmung der Grund-Aktivität (Zyklus 0) insgesamt zweimal wiedergewonnen und mit frischem Substrat versetzt (Zyklus 1 und 2). Die prozentuale Aktivität der recycelten Cellulase-Stämme wurde durch Vergleich mit der jeweiligen Grund-Aktivität ermittelt.

### Recycling der β-Glucosidase Bgla: pNPGase-Aktivität

Die β-Glucosidase Aktivität wurde mit pNitrophenol-Glucopyranosid (pNPG) über die Zunahme des pNitrophenolats bei 400 nm photometrisch bestimmt (pNPGase-Aktivität). Dazu wurde der BglA-Stamm (OD₅₇₈20) mit pNPG für 15 min bei pH 6 und 60°C inkubiert. Anschießend wurden die Zellen sedimentiert und die Aktivität nach Resuspension emeut gemessen (zyklus 1 und 2). Die Aktivität wurde über die Zunahme des p-Nitrophenolats bei 400 nm bestimmt, und stellt einen Mittelwert aus drei Einzelmessungen dar. Das Ergebnis der Recyclierungversuche zeigt, dass sich die Enzymaktivität im Rahmen der Standardabweichung für alle Zyklen auf gleichem Niveau bewegt Die Recyclierungen hatten damit keinen Effekt auf die BlgA-Aktivität. Der BglA-Stamm kann somit mindestens zweimal nach Rückgewinnung aus dem Reaktionsansatz wiederverwendet werden.

### Recycling der Exocellulase CelK: pNPCase-Aktivität

Die Aktivität der Exocellulase wurde mit pNitrophenol-Cellobiosid (pNPC) über die Zunahme von pNitrophenolat bei 400 nm photometrisch bestimmt (pNPCase-Aktivität). Dazu wurde der CelK-Stamm (OD₅₇₈0,5) mit pNPC für 4 min bei pH 6 und 60°C inkubiert. Anschließend wurden die Zellen sedimentiert und die Aktivität nach Resuspension erneut gemessen (Zyklus 1 und 2). Die Aktivität wurde über die Zunahme des p-Nitropnenolats bei 400 nm bestimmt, und stellt einen Mittelwert aus drei Einzeimessungen dar. Das Ergebnis der Recyclierungversuche zeigt, dass sich die Enzymaktivität im Rahmen der Standardabweichung für alle Zyklen auf gleichem Niveau bewegt. Die Recyclierungen hatten darmit keinen Effekt auf die CelK-Aktivität Entsprechend dem BglA-Stamm kann auch der CelK-Stamm mindestens zweimal wiederverwendet werden.

### Recycling der Endocellulase Cel5A: CMCase-Aktivität

Die Endocellulase-Aktivität wurde mit CMC über die Bestimmung der red. Zucker mittels DNS-Assay ermittelt (CMCase-Aktivität). Dazu wurde der Cel5A-Stamm (OD₅₇₈20) mit CMC für 15 min bei pH 6 und 60°C inkubiert, Anschließend wurden die Zellen sedimentiert und die Aktivität nach Resuspension erneut bestimmt (Zyklus 1 und 2). Die freigesetzten reduzierenden Zucker wurden mittels DNS-Assay bei 540 nm detektiert Die bestimmten Werte sind Mittelwerte aus drei Einzelmessungen. Die Cel5A-Aktivität weist nach der ersten Rückgewinnung eine Abnahme von ca. 14 % auf (Zyklus 1). Nach einer erneuten Recyclierung (Zyklus 2) ist eine Aktivitäts-Abnahme von ca. 63 % zu verzeichnen, verglichen mit der Grund-Aktivität (Zyklus 0). Im Gegensatz zum BglA- und CelK- Stamm geht die Recyclierung des Cel5A-Stamms mit einer Aktivitäts-Verminderung einher. Aufgrund der moderaten Aktivitäts-Abnahme nach dem ersten Zyklus kann der Cel5A-Stamm aber mindestens einmal wiederverwendet werden.

### Beispiel 7: Filter Papier Assay (FPA)

Der FPA ist die Standard methode für die Analyse der gesamten Cellulase-Aktivität (Exo-, Endocellulase sowie B-Glucosidase) und wurde von Mandels et al 1976 entwickelt. Nach IUPAC (International Union of Pure and Applied Chemistry) wird der FPA mit einem 1x6 cm Filterpapier Streifen (Whatman Nr 1) als Standardsubstrat (ca. 50 mg) und 1,5 ml Gesamtvolumen durchgeführt. Die internationale Einhelt der Filterpapieraktivitat (FPU) ist definiert als mikromol Glucose-Äquivalente (reduzierende Zucker), die pro Minute freigesetzt werden. Mit dem FPA sollten nicht mehr als 2 mg Glucose aus 50 mg Filterpapier (4 % Umsatz) in 60 min freigesetzt werden. Aufgrund der Struktur der Cellulose ist der Abbau nicht linear. Daher wird nur ein Urnsatz bis 4 % als linear beschrieben. Die gebildeten reduzierenden Zucker werden mit Hilfe der DNS-Methode bestimmt, Da nun für jede Autodisplay-Cellulase Aktivität nachgewiesen werden konnte, sollte nun die synergistische Aktivität untersucht werden. Dazu wurde ein Mix aus allen drei Cellulasen hergestellt und die FP-Aktivität ermittelt.

### FP-Assay im Mikrotiterplatten-MaBstab

Der FPA nach IUPAC ist aufgrund des Gesamtvolumens von 1,5 ml und der hohen Mengen an einzusetzenden DNS-Reagenz (3 ml pro Ansatz) umständlich, wenn man mehrere Cellulase-Mixe auf FP-Aktivität hin untersuchen möchte, In der Literatur ist der FP-Assay im Mikrotiterplatten-MaBstab mit einem Gesamtvolumen von 71 µl beschrieben (Xiao 7 et al, 2004). Dazu wurden FP-Plättchen mit Hilfe eines Lochers hergestellt. Das Verhältnis von Reaktionsvolumen zu Filterpapier wurde über die Filterpapier-Fläche (FP-Plättchen vs. FP-Streifen) berechnet. So wurde gewährleistet dass auch im Mikrotitierplatten-Maßstab der Ansatz gemäß IUPAC erfolgte. Da nicht bekannt war, welches das beste Mischungsverhältnis der einzelnen Autodisplay-Cellulasen ist, wurden verschiedene Ansätze auf FP-Aktivität hin getestet. Ansätze ohne BglA-Stamm wurden mit B-Glucosidase aus Mandel versetzt. Als Kontrolle diente der Wirtsstamm (8Z) und FP-Plättchen mit Puffer (BS). Die FPase-Aktivität wurde nach unterschiedlichen Inkubetiortszeitien bei 60°c über die freigesetzten reduzierenden Zucker mittels DNS-Assay bestimmt. Vor der Messung wurden alle Ansätze für 10 min mit B-Glucosidase versetzt, um sicher zu stellen, dass die noch nicht umgesetzte Cellobiose in Glucose abgebaut wird, was eine genauere Bestimmung der reduzierenden Zucker zur Folge hat. Nach Abtrennung der Zellen wurden die reduzierenden Zucker im Überstand bei 540 nm bestimmt. Das Ergebnis ist in Figur 20 dargestellt Sowohl der Cellulase-Mix mit kommerztell erhältlicher B-Glucosidase als auch der Ansatz mit allen drei Autodisplay-Cellulasen zeigten eine Umsetzung des Filterpaplers. Nach 5 Tagen Inkubation bei 60°C erwies sich der 50:50 (CelK-Cel5A)-Mix am besten. Die eingesetzten Kontrollen waren negativ und zeigten keinen Umsatz des Filterpapier (Figur 20A). Mit Hilfe der Glukose-Standardkurve wurde die Menge der freigesetzten reduzierenden Zucker in mg Glucose/ml ermittelt (vgl. Figur 20B). Nach 5 Tagen konnten ca. 0,2 mg/ml erzielt werden. Aufgrund der amorphen und kristallinen Struktur der Cellulose ist der Abbau keine lineare Reaktion. Zur Bestimmung der FPase-Aktivität in FPU/I wurden daher, wie nach IUPAC angegeben, die 1 h Messwerte herangezogen. Das Ergebnis ist in Tabelle 6 zusammengefasst.

**Tabelle 6: .**

| CelK:Cel5A:BglA | FPU/I |
|---|---|
| 30:70:0 | 2,01 |
| 50:50:0 | 1,91 |
| 70:30:0 | 2,45 |
| 50:25:25 | 0,90 |

### FP-Assay Standard

Aufgrund des geringen Gesamtvolumens (71 µl), welches für die Umsetzung des FP-Plättchens eingesetzt wurde, erwies sich die Abtrennung der Zellen nach 3 Tagen als schwierig, da das FP-Plättchen fast die komplette Flüssigkeit aufgesogen hatte. Daher sollte der Standard FP-Assay nach IUPAC mit einem Gesamtvolumen von 1,5 ml durchgeführt werden. Als Vergleich dienten ein Ansatz im Mikrotiterplatten-MaBstab. Dazu wurden alle drei Autodisplay-Cellulasen im gleichen Verhältnis gemischt und zum einen mit einem 1x6 cm Filterpapier-Streifen (standard) und zum anderen mik einem Filterpapier-Plättchen (Mikro) 3 Tage bei 60°C inkubiert. Als Kontrolle diente der Wirtsstamm und Filterpapier ohne Zeilen (BS). Das Ergebnis ist in Figur 21 dargesteilt. Es ist zu erkennen, dass mehr reduzierende Zucker im Standard-Ansatz nach 3 Tagen bei 60°C gebildet werden, verglichen mit dem Mikro-Ansatz. Im Standard-Ansatz ist die Durchmischung besser, da die Zellen immer in Kontakt mit dem Filterpape-Streifen sind. Im Mikro-Ansatz befindet sich ein Großteil der Zellen Oberhalb des Filterpapier-Plättchens, sodass nicht immer alle Zellen mit dem FP in Kontakt sind. Die beiden eingesetzten Kontrollen zeigten wie erwartet keine FPase-Aktivität (Figur 21A). Mit Hilfe der Glukose-Standardkurve wurde die Menge der freigesetzten reduzierenden Zucker in mg Glucose/ml ermittelt (vgl. Figur 21B). Im Mikro-Ansatz konnten nach 3 Tagen bei 60°C 0,06 mg/ml reduzierenden Zucker ermittelt werden. Im Gegensatz dazu wurden im Standard-Ansatz 0,38 mg/ml erreicht werden. Durch eine bessere Durchmischung konnte daher eine 6,3fach verbesserte Ausbeute erzielt werden.

### Beispiel 8: Umsetzung von Pappelholz

Es konnte gezeigt werden, dass die Autudisplay-Cellulasen Filterpapier abbauen können (vgl. Figur 20). Da Filterpape kein Lignin und nur einen sehr geringen Anteil an Hemlcelulose enthält, solite nun ein komplexeres Substrat verwendet werden. Dafür wurde mit Dampfdruck behandelte Pappel eingesetzt. Durch die Vorbehandlung wurde die Hemicellulose entfernt, wohingegen Lignin noch vorhanden ist. Lignin bildet zum einen eine physikatische Bardere, da es die Angriffsfläche der Cellulasen bedenkt und zum anderen eine chemische Barriere, da Cellulasen von Lignin gebunden werden können und somit ebenfalls nicht mehr an die Cellulose gelangen können. Um den Pappet-Abbau der Cellulasen zu untersuchen, wurden die Autodisplay-Cellulasen zu gleichen Teilen gemischt und mit 4 % Pappel für 88 h bei 60°C lnkubiert. Als Kontrolle dienten der Wirtsstamm und Pappel in Puffer ohne Zellen (BS). Die Pappel und die Zellen wurden nach Inkubation durch Zentrifugation sedimentiert. Der Überstand wurde weiter verwendet, um gebildete reduzierende Zucker mittels DNS-Assay zu bestimmen. Das Ergebnis ist in Figur 22 dargestellt. Die Überstände wiesen schon vor der Messung eine gelbliche Farbe auf, ähnlich dem DNS-Reagenz. Daher sind nach der Inkubation mit dem DNS-Reagenz die Absorptlonswerte bei 540 nm höher verglichen mit denen vom FP-Assay, bel dem die Überstände klar sind (vgl. Figur 20). Es war nicht möglich die Konzentration in mg Glucose/mi zu ermitteln, da keine adäquaten Glukose-Standards vorlagen. Michtsdestotrotz konnte mit dem Cellulasen eine höhere Absorption nach 88 h bei 60°C beobachtet werden, verglichen mit den beiden eingesetzten Kontrollen. Die Autodisplay-Cellulasen sind somit in der Lage auch komplekere Substrate umzusetzen.

### Literatur:

Jose und Meyer (2007), Microbiol Mol Biol Rev 71 (4), 600-19,
Jose (2006), Appl. Microbiol. Biotechnol. 2006, 69, 607-614.
Jose etal (2002), J. Blotechnol. 2002, 95, 257-268.
lose etal. (1995), Mol. Microbiol., (2), 378-80.
Ausubel etal. (1995), Current Protocols in Molecular Biology, Wiley Interscience Pubitshers.
Henderson etal. (2004), Microbiol, and Molecular Biology Reviews, 68(4), 692-744.
Jose und von Schwichow (2004), ChemBtoChem, 5, 491-499,
Banerjee et al. (2002), Appl. Microblol, Biotechnol. 2002, 60, 33-44.
Niewert et al. (2001) Diarrhea. Clin. Diagn. Lab. Immunol. (1): 143-149;9.
Lin L, Meng X, Llu P, Hong Y et al (2009) Appl Microblol Biotechnol 82: 671-679
Miller GL (1959) Analytical Chemistry 31:426-428
Xiao Zhizhuang, Storms R, Tsang A (2004) Biotechnology and Bioengineering 88 (7): 832-837

## Patentansprüche

1. Nukleinsäuremolekül, umfassend die folgenden Bestandteile:
(1) einen Abschnitt, der ein Signalpeptid codiert,
(2) einen Abschnitt, der eine heterologe Cellulase codiert,
(3) optional einen Abschnitt, der eine Protease-Erkennungsstelle codiert,
(4) einen Abschnitt, der einen Transmembranlinker codiert, und
(5) einen Abschnitt, der eine Transporterdomäne eines Autotransporters oder einer Variante davon codiert.

2. Nukleinsäuremolekül gemäß Anspruch 1, **dadurch gekennzeichnet, dass** die Cellulase eine beta-Glucosidase oder eine Endo- oder Exocellulase ist, vorzugsweise ausgewählt aus der Gruppe umfassend *Bacillus subtilis* Endocellulase, *Clostridium thermocellum* Exocellulase und *Clostridicrm thermocellum* beta-Glucosidase.

3. Nukleinsäuremolekül gemäß Anspruch 1 oder 2, **dadurch gekennzeichnet, dass** die Transporterdomäne eines Autotransporters ausgewählt ist aus der Gruppe, umfassend Ssp, Ssp-h1, Ssp-h2, PspA, PspB, Ssa1, SphB1, AspA/NalP, VacA, AIDA-I, IcsA, MisL, TibA, Ag43, ShdA, AutA, Tsh, SepA, EspC, EspP, Pet, Pic, SigA, Sat, Vat, EpeA, EatA, EspI, EaaA, EaaC, Pertactin, BrkA, Tef, Vag8, PmpD, Pmp20, Pmp21, AgA1 Protease, App, Hap, rOmpA, rOmpB, ApeE, EstA, Lip-1, McaP, BabA, SabA, AlpA, Aae, NanB und Varianten davon.

4. Nukleinsäuremolekül gemäß einem der Ansprüche 1 bis 3, **dadurch gekennzeichnet, dass** es eine operativ an das Nukleinsäuremolekül gebundene Expressionskontrollsequenz enthält, die vorzugsweise durch Zugabe von Isopropylthioglucopyranosid (IPTG) oder Arabinose aktivierbar ist.

5. Polypeptid, codiert durch ein Nukleinsäuremolekül nach einem der Ansprüche 1 bis 4.

6. Mikroorganismus, der an seiner Oberfläche ein Polypeptid nach Anspruch 5 exprimiert oder unter Verwendung eines Nukleinsäuremoleküls nach einem der Ansprüche 1 bis 4 transformiert worden ist.

7. Mikroorganismus nach Anspruch 6, **dadurch gekennzeichnet, dass** er auf einem Gramnegativen Mikroorganismus, vorzugsweise Escherichia coli beruht.

8. Membranfraktion, erhältlich von der Zelle nach Anspruch 6 oder 7.

9. Verfahren zur Herstellung eines Reaktionsprodukts mit Hilfe von mindestens einer Cellulase, umfassend die folgenden Schritte:
(i) Bereitstellen eines Mikroorganismus nach Anspruch 6 oder 7 oder eines Membranpräparats nach Anspruch 8, und
(ii) Inkontaktbringen des Mikroorganismus und/oder des Membranpräparats mit ein oder mehreren Cellulase Substraten unter Bedingungen, die mit der Cellulase Aktivität kompatibel sind.

10. Verfahren nach Anspruch 9, **dadurch gekennzeichnet, dass** das Produkt der durch die Cellulase katalysierten Reaktion ein Mono-, Di- oder Oligosaccharid ist, und dass das mindestens eine Cellulase-Substrat eine Polysaccharid-Quelle, vorzugsweise eine CelluloseQuelle ist.

11. Verfahren nach Anspruch 9 oder 10, **dadurch gekennzeichnet, dass** der Schritt (ii) bei einem pH im Bereich von 4,5 bis 6,5, vorzugsweise im Bereich von 5,5 bis 6,5 durchgeführt wird.

12. Verfahren nach einem der Ansprüche 9 bis 11, **dadurch gekennzeichnet, dass** der Schritt (ii) bei einer Temperatur im Bereich von 30 bis 80 °C, vorzugsweise im Bereich von 50 bis 65 °C durchgeführt wird.

13. Verfahren nach einem der Ansprüche 9 bis 12, **dadurch gekennzeichnet, dass** im Schritt (ii) eine Glucosidase zugesetzt wird.

14. Verfahren nach einem der Ansprüche 9 bis 13, **dadurch gekennzeichnet, dass** es zusätzlich einen Schritt (iii) des Zurückgewinnens des in Schritt (ii) verwendeten Mikroorganismus umfasst.

15. Verfahren zum Herstellen eines Mikroorganismus, der an seiner Oberfläche eine rekombinante Cellulase präsentiert, umfassend
a) das Einbringen einer Nukleinsäuresequenz gemäß einem der Ansprüche 1 bis 4 in den Mikroorganismus, und
b) optional das Behandeln des Mikroorganismus mit einer Substanz, die die Expressionskontrollsequenz aktiviert.


SEQUENCE LISTING

<110> Zyrus Beteiligungsgesellschaft mbH & Co. Patente I KG

<120> Ganzell-Biokatalysatoren zum Abbau von Cellulosischer Biomasse

<130> IPB-227-DE

<160> 6

<170> BiSSAP 1.2

<210> 1
<211> 1422
<212> DNA
<213> Artificial Sequence

<220>
<221> source
<222> 1..1422
<223> /organism="Artificial Sequence"
/note="optimierte Endocellulase Cel5A Gensequenz aus Bacillus
subtilis"
/mol_type="unassigned DNA"

<400> 1
ctcgaggcag gcaccaaaac accggttgca aaaaatggtc agctgagtat taaaggcacc 60

cagctggtta atcgtgatgg taaagcagtg cagctgaaag gtattagcag ccatggtctg 120

cagtggtatg gtgaatttgt taataaagac agcctgaaat ggctgcgtga tgattggggt 180

attaccgttt ttcgtgcagc aatgtatacc gcagatggtg gctatattga taatccgagc 240

gttaaaaaca aagtgaaaga agcagttgaa gcagccaaag aactgggcat ttatgtgatt 300

attgattggc acattctgaa cgatggtaat ccgaatcaga acaaagaaaa agcgaaagaa 360

tttttcaaag aaatgagcag cctgtatggc aataccccga atgtgattta tgaaattgcc 420

aatgaaccga atggtgatgt gaattggaaa cgcgatatca aaccgtatgc cgaagaagtg 480

attagcgtta ttcgtaaaaa tgacccggac aacattatta tcgttggcac cggcacctgg 540

tcacaggatg ttaatgatgc agcagatgat caactgaaag atgccaatgt tatgtatgcc 600

ctgcattttt atgcaggcac ccatggtcag tttctgcgtg ataaagcaaa ttatgcactg 660

agcaaaggtg caccggtttt tgttaccgaa tggggcacca gtgatgcaag cggtaatggt 720

ggtgtttttc tggatcagag ccgtgaatgg ctgaattatc tggatagcaa aaccattagc 780

tgggtgaact ggaatctgag cgataaacaa gaaagcagca gcgcactgaa accgggtgca 840

agcaaaacag gtggttggcg tctgagtgat ctgagcgcaa gcggcacctt tgttcgtgaa 900

aatattctgg gtacaaaaga tagcaccaaa gacattccgg aaacaccggc aaaagataaa 960

ccgacccaag aaaatggtat tagcgttcag tatcgtgccg gtgatggtag catgaatagc 1020

aatcagattc gtccgcagct gcagatcaaa aacaatggta ataccaccgt ggatctgaaa 1080

gacgttaccg cacgttattg gtataacgcc aaaaacaaag gccagaatgt ggattgtgat 1140

tatgcacagc tgggttgtgg taatatgacc cataaatttg tgacactgca caaaccgaaa 1200

cagggtgcag atacctatct ggaactgggt ttcaaaaatg gcaccctggc accgggtgcc 1260

tcaaccggta atattcagct gcgtctgcat aacgatgatt ggagcaatta tgcccagagc 1320

ggtgattata gctttttcaa aagcaacacc tttaaaacca ccaaaaaaat caccctgtat 1380

gaccagggta aactgatttg gggcaccgaa ccgaacggta cc 1422

<210> 2
<211> 474
<212> PRT
<213> Artificial Sequence

<220>
<223> Aus Seq_1 abgeleitete Endocellulase Cel5A Peptisequenz

<400> 2
Leu Glu Ala Gly Thr Lys Thr Pro Val Ala Lys Asn Gly Gln Leu Ser
1               5                   10                  15
Ile Lys Gly Thr Gln Leu Val Asn Arg Asp Gly Lys Ala Val Gln Leu
            20                  25                  30
Lys Gly Ile Ser Ser His Gly Leu Gln Trp Tyr Gly Glu Phe Val Asn
        35                  40                  45
Lys Asp Ser Leu Lys Trp Leu Arg Asp Asp Trp Gly Ile Thr Val Phe
    50                  55                  60
Arg Ala Ala Met Tyr Thr Ala Asp Gly Gly Tyr Ile Asp Asn Pro Ser
65                  70                  75                  80
Val Lys Asn Lys Val Lys Glu Ala Val Glu Ala Ala Lys Glu Leu Gly
                85                  90                  95
Ile Tyr Val Ile Ile Asp Trp His Ile Leu Asn Asp Gly Asn Pro Asn
            100                 105                 110
Gln Asn Lys Glu Lys Ala Lys Glu Phe Phe Lys Glu Met Ser Ser Leu
        115                 120                 125
Tyr Gly Asn Thr Pro Asn Val Ile Tyr Glu Ile Ala Asn Glu Pro Asn
    130                 135                 140
Gly Asp Val Asn Trp Lys Arg Asp Ile Lys Pro Tyr Ala Glu Glu Val
145                 150                 155                 160
Ile Ser Val Ile Arg Lys Asn Asp Pro Asp Asn Ile Ile Ile Val Gly
                165                 170                 175
Thr Gly Thr Trp Ser Gln Asp Val Asn Asp Ala Ala Asp Asp Gln Leu

180                 185                 190
Lys Asp Ala Asn Val Met Tyr Ala Leu His Phe Tyr Ala Gly Thr His
        195                 200                 205
Gly Gln Phe Leu Arg Asp Lys Ala Asn Tyr Ala Leu Ser Lys Gly Ala
    210                 215                 220
Pro Val Phe Val Thr Glu Trp Gly Thr Ser Asp Ala Ser Gly Asn Gly
225                 230                 235                 240
Gly Val Phe Leu Asp Gln Ser Arg Glu Trp Leu Asn Tyr Leu Asp Ser
                245                 250                 255
Lys Thr Ile Ser Trp Val Asn Trp Asn Leu Ser Asp Lys Gln Glu Ser
            260                 265                 270
Ser Ser Ala Leu Lys Pro Gly Ala Ser Lys Thr Gly Gly Trp Arg Leu
        275                 280                 285
Ser Asp Leu Ser Ala Ser Gly Thr Phe Val Arg Glu Asn Ile Leu Gly
    290                 295                 300
Thr Lys Asp Ser Thr Lys Asp Ile Pro Glu Thr Pro Ala Lys Asp Lys
305                 310                 315                 320
Pro Thr Gln Glu Asn Gly Ile Ser Val Gln Tyr Arg Ala Gly Asp Gly
                325                 330                 335
Ser Met Asn Ser Asn Gln Ile Arg Pro Gln Leu Gln Ile Lys Asn Asn
            340                 345                 350
Gly Asn Thr Thr Val Asp Leu Lys Asp Val Thr Ala Arg Tyr Trp Tyr
        355                 360                 365
Asn Ala Lys Asn Lys Gly Gln Asn Val Asp Cys Asp Tyr Ala Gln Leu
    370                 375                 380
Gly Cys Gly Asn Met Thr His Lys Phe Val Thr Leu His Lys Pro Lys
385                 390                 395                 400
Gln Gly Ala Asp Thr Tyr Leu Glu Leu Gly Phe Lys Asn Gly Thr Leu
                405                 410                 415
Ala Pro Gly Ala Ser Thr Gly Asn Ile Gln Leu Arg Leu His Asn Asp
            420                 425                 430
Asp Trp Ser Asn Tyr Ala Gln Ser Gly Asp Tyr Ser Phe Phe Lys Ser
        435                 440                 445
Asn Thr Phe Lys Thr Thr Lys Lys Ile Thr Leu Tyr Asp Gln Gly Lys
    450                 455                 460
Leu Ile Trp Gly Thr Glu Pro Asn Gly Thr
465                 470

<210> 3
<211> 2382
<212> DNA
<213> Artificial Sequence

<220>
<221> source
<222> 1..2382
<223> /organism="Artificial Sequence"
      /note="Optimierte Gensequenz einer Exocellulase CelK aus
      Clostridium thermocellum"
      /mol_type="unassigned DNA"

<400> 3
ctcgagctgg aagataaaag cagcaaactg ccggattaca aaaacgatct gctgtatgaa      60

cgtacctttg atgaaggtct gtgttttccg tggcatacct gtgaagatag cggtggtaaa 120

tgtgattttg ccgttgttga tgttccgggt gaaccgggta ataaagcatt tcgtctgacc 180

gttattgata aaggccagaa taaatggtca gtgcagatgc gtcatcgtgg tattaccctg 240

gaacagggtc atacctatac cgttcgtttt accatttgga gcgataaaag ctgtcgtgtg 300

tatgcaaaaa ttggtcagat gggcgaaccg tataccgaat attggaataa taactggaac 360

ccgtttaatc tgacccctgg tcagaaactg accgttgaac agaattttac catgaactat 420

ccgaccgatg atacctgcga attcaccttt catctgggtg gtgaactggc agcaggcacc 480

ccgtattatg tttatctgga tgatgttagc ctgtacgatc cgcgttttgt taaaccggtt 540

gaatatgttc tgccgcagcc ggatgttcgt gttaatcagg ttggttatct gccgtttgcc 600

aaaaaatacg caaccgttgt tagcagcagc accagtccgc tgaaatggca gctgctgaat 660

agcgcaaatc aggtggttct ggaaggcaat accattccga aaggtctgga taaagatagc 720

caggattatg tgcattggat cgatttcagc aactttaaaa ccgaaggcaa aggctactac 780

tttaaactgc cgaccgttaa tagcgatacc aattatagcc atccgtttga tattagcgca 840

gatatctata gcaaaatgaa atttgatgcc ctggccttct tttatcataa acgtagcggt 900

attccgatcg aaatgccgta tgcaggcggt gaacagtgga cccgtccggc aggtcatatt 960

ggtattgaac cgaataaagg tgataccaat gttccgacct ggcctcagga tgatgaatat 1020

gcaggtcgtc cgcagaaata ttacaccaaa gatgttaccg gtggttggta tgatgccggt 1080

gatcatggca aatatgttgt gaatggtggt attgcagttt ggaccctgat gaacatgtat 1140

gagcgtgcca aaattcgcgg tattgccaat cagggtgcat ataaagatgg tggcatgaat 1200

attccggaac gcaataatgg ttatccggac attctggatg aagcacgttg ggaaattgaa 1260

tttttcaaaa aaatgcaggt taccgaaaaa gaagatccga gcattgcagg tatggtgcat 1320

cataaaattc atgattttcg ttggaccgca ctgggtatgc tgccgcatga agatccgcag 1380

cctcgttatc tgcgtccggt tagcaccgca gcaaccctga attttgcagc caccctggca 1440

cagagcgcac gtctgtggaa agattatgat ccgacctttg cagcagattg tctggaaaaa 1500

gcagaaattg catggcaggc agcactgaaa catccggata tttatgcaga atatacaccg 1560

ggtagtggtg gtccgggtgg cggtccgtat aatgatgatt atgttggtga tgagttttat 1620

tgggcagcct gtgaactgta tgttaccacc ggcaaagatg agtataaaaa ctatctgatg 1680

aactcaccgc actacctgga aatgcctgca aaaatgggtg aaaatggtgg tgcaaatggt 1740

gaagataatg gtctgtgggg ttgttttacc tggggtacaa cccagggtct gggcaccatt 1800

acactggcac tggtggaaaa tggtctgcct gcaaccgata ttcagaaagc acgtaataac 1860

attgcaaaag cagccgatcg ttggctggaa aatattgaag aacagggtta tcgtctgccg 1920

attaaacagg ccgaagatga acgtggtggc tatccgtggg gtagcaatag ctttattctg 1980

aatcagatga tcgtgatggg ctatgcctat gattttaccg gcaatagcaa atatctggac 2040

ggtatgcagg atggtatgag ctatctgctg ggtcgcaatg gtctggatca gagctatgtg 2100

accggttatg gtgaacgtcc gctgcagaat ccgcatgatc gcttttggac accgcagacc 2160

agcaaaaaat tccctgcacc gcctccgggt attattgcgg gtggtccgaa tagccgtttt 2220

gaagatccta ccattaccgc agcagtgaaa aaagataccc ctcctcagaa atgctatatc 2280

gatcataccg atagctggtc caccaatgaa attaccgtta attggaatgc accgtttgca 2340

tgggttaccg cgtacctgga tgaaattgat ctgattggta cc 2382

<210> 4
<211> 794
<212> PRT
<213> Artificial Sequence

<220>
<223> Aus Seq_3 abgeleitete Peptidsequenz con Exocellulase CelK

<400> 4
   Leu Glu Leu Glu Asp Lys Ser Ser Lys Leu Pro Asp Tyr Lys Asn Asp
   1 5 10 15
   Leu Leu Tyr Glu Arg Thr Phe Asp Glu Gly Leu Cys Phe Pro Trp His
   20 25 30
   Thr Cys Glu Asp Ser Gly Gly Lys Cys Asp Phe Ala Val Val Asp Val
   35 40 45
   Pro Gly Glu Pro Gly Asn Lys Ala Phe Arg Leu Thr Val Ile Asp Lys
   50 55 60
   Gly Gln Asn Lys Trp Ser Val Gln Met Arg His Arg Gly Ile Thr Leu
   65 70 75 80
   Glu Gln Gly His Thr Tyr Thr Val Arg Phe Thr Ile Trp Ser Asp Lys
   85 90 95
   Ser Cys Arg Val Tyr Ala Lys Ile Gly Gln Met Gly Glu Pro Tyr Thr
   100 105 110
   Glu Tyr Trp Asn Asn Asn Trp Asn Pro Phe Asn Leu Thr Pro Gly Gln
   115 120 125
   Lys Leu Thr Val Glu Gln Asn Phe Thr Met Asn Tyr Pro Thr Asp Asp
   130 135 140
   Thr Cys Glu Phe Thr Phe His Leu Gly Gly Glu Leu Ala Ala Gly Thr
   145 150 155 160
   Pro Tyr Tyr Val Tyr Leu Asp Asp Val Ser Leu Tyr Asp Pro Arg Phe
   165 170 175
   Val Lys Pro Val Glu Tyr Val Leu Pro Gln Pro Asp Val Arg Val Asn
   180 185 190
   Gln Val Gly Tyr Leu Pro Phe Ala Lys Lys Tyr Ala Thr Val Val Ser
   195 200 205
   Ser Ser Thr Ser Pro Leu Lys Trp Gln Leu Leu Asn Ser Ala Asn Gln
   210 215 220
   Val Val Leu Glu Gly Asn Thr Ile Pro Lys Gly Leu Asp Lys Asp Ser
   225 230 235 240
   Gln Asp Tyr Val His Trp Ile Asp Phe Ser Asn Phe Lys Thr Glu Gly
   245 250 255
   Lys Gly Tyr Tyr Phe Lys Leu Pro Thr Val Asn Ser Asp Thr Asn Tyr
   260 265 270
   Ser His Pro Phe Asp Ile Ser Ala Asp Ile Tyr Ser Lys Met Lys Phe
   275 280 285
   Asp Ala Leu Ala Phe Phe Tyr His Lys Arg Gly Ser Ile Pro Ile Glu
   290 295 300
   Met Pro Tyr Ala Gly Gly Glu Gln Trp Thr Arg Pro Ala Gly His Ile
   305 310 315 320
   Gly Ile Glu Pro Asn Lys Gly Asp Thr Asn Val Pro Thr Trp Pro Gln
   325 330 335
   Asp Asp Glu Tyr Ala Gly Arg Pro Gln Lys Tyr Tyr Thr Lys Asp Val
   340 345 350
   Thr Gly Gly Trp Tyr Asp Ala Gly Asp His Gly Lys Tyr Val Val Asn
   355 360 365
   Gly Gly Ile Ala Val Trp Thr Leu Met Asn Met Tyr Glu Arg Ala Lys
   370 375 380
   Ile Arg Gly Ile Ala Asn Gln Gly Ala Tyr Lys Asp Gly Gly Met Asn
   385 390 395 400
   Ile Pro Glu Arg Asn Asn Gly Tyr Pro Asp Ile Leu Asp Glu Ala Arg
   405 410 415
   Trp Glu Ile Glu Phe Phe Lys Lys Met Gln Val Thr Glu Lys Glu Asp
   420 425 430
   Pro Ser Ile Ala Gly Met Val His His Lys Ile His Asp Phe Arg Trp
   435 440 445
   Thr Ala Leu Gly Met Leu Pro His Glu Asp Pro Gln Pro Arg Tyr Leu
   450 455 460
   Arg Pro Val Ser Thr Ala Ala Thr Leu Asn Phe Ala Ala Thr Leu Ala
   465 470 475 480
   Gln Ser Ala Arg Leu Trp Lys Asp Tyr Asp Pro Thr Phe Ala Ala Asp
   485 490 495
   Cys Leu Glu Lys Ala Glu Ile Ala Trp Gln Ala Ala Leu Lys His Pro
   500 505 510
   Asp Ile Tyr Ala Glu Tyr Thr Pro Gly Ser Gly Gly Pro Gly Gly Gly
   515 520 525
   Pro Tyr Asn Asp Asp Tyr Val Gly Asp Glu Phe Tyr Trp Ala Ala Cys
   530 535 540
   Glu Leu Tyr Val Thr Thr Gly Lys Asp Glu Tyr Lys Asn Tyr Leu Met
   545 550 555 560
   Asn Ser Pro His Tyr Leu Glu Met Pro Ala Lys Met Gly Glu Asn Gly
   565 570 575
   Gly Ala Asn Gly Glu Asp Asn Gly Leu Trp Gly Cys Phe Thr Trp Gly
   580 585 590
   Thr Thr Gln Gly Leu Gly Thr Ile Thr Leu Ala Leu Val Glu Asn Gly
   595 600 605
   Leu Pro Ala Thr Asp Ile Gln Lys Ala Arg Asn Asn Ile Ala Lys Ala
   610 615 620
   Ala Asp Arg Trp Leu Glu Asn Ile Glu Glu Gln Gly Tyr Arg Leu Pro
   625 630 635 640
   Ile Lys Gln Ala Glu Asp Glu Arg Gly Gly Tyr Pro Trp Gly Ser Asn
   645 650 655
   Ser Phe Ile Leu Asn Gln Met Ile Val Met Gly Tyr Ala Tyr Asp Phe
   660 665 670
   Thr Gly Asn Ser Lys Tyr Leu Asp Gly Met Gln Asp Gly Met Ser Tyr
   675 680 685
   Leu Leu Gly Arg Asn Gly Leu Asp Gln Ser Tyr Val Thr Gly Tyr Gly
   690 695 700
   Glu Arg Pro Leu Gln Asn Pro His Asp Arg Phe Trp Thr Pro Gln Thr
   705 710 715 720
   Ser Lys Lys Phe Pro Ala Pro Pro Pro Gly Ile Ile Ala Gly Gly Pro
   725 730 735
   Asn Ser Arg Phe Glu Asp Pro Thr Ile Thr Ala Ala Val Lys Lys Asp
   740 745 750
   Thr Pro Pro Gln Lys Cys Tyr Ile Asp His Thr Asp Ser Trp Ser Thr
   755 760 765
   Asn Glu Ile Thr Val Asn Trp Asn Ala Pro Phe Ala Trp Val Thr Ala
   770 775 780
   Tyr Leu Asp Glu Ile Asp Leu Ile Gly Thr
   785 790

<210> 5
<211> 1353
<212> DNA
<213> Artificial Sequence

<220>
<221> source
<222> 1..1353
<223> /organism="Artificial Sequence"
/note="optimierte Gensequenz der beta-Glucosidase BglA aus
Clostridium thermocellum"
/mol_type="unassigned DNA"

<400> 5
ctcgagagca aaattacctt cccgaaagat tttatctggg gtagcgcaac cgcagcatat 60

cagattgaag gtgcatataa tgaagatggc aaaggcgaaa gcatttggga tcgttttagc 120

catacaccgg gtaatattgc agatggtcat accggtgatg ttgcctgtga tcattatcat 180

cgttatgaag aggatatcaa aatcatgaaa gagatcggca tcaaaagcta tcgctttagc 240

attagctggc ctcgtatttt tccggaaggc accggtaaac tgaatcagaa aggtctggat 300

ttctataaac gtctgaccaa tctgctgctg gaaaatggta ttatgcctgc aattaccctg 360

tatcattggg atctgccgca gaaactgcag gataaaggtg gttggaaaaa tcgtgatacc 420

accgattatt tcaccgaata tagcgaggtg atctttaaaa acctgggtga tattgtgccg     480

atctggttta cccataatga accgggtgtt gttagcctgc tgggtcattt tctgggtatt     540

catgcaccgg gtattaaaga tctgcgtacc agcctggaag ttagccataa cctgctgctg     600

agccatggta aagcagttaa actgtttcgc gagatgaata ttgatgccca gattggtatt     660

gcactgaacc tgagctatca ttatccggca agcgaaaaag cagaagatat tgaagcagca     720

gaactgagct ttagcctggc aggtcgttgg tatctggatc cggttctgaa aggtcgttat     780

ccggaaaatg cactgaaact gtacaaaaaa aaaggcatcg aactgtcgtt cccggaagat     840

gatctgaaac tgattagcca gccgattgat tttatcgcct ttaacaatta tagcagcgag     900

ttcatcaaat atgatccgag cagcgaaagc ggttttagtc cggcaaatag cattctggaa     960

aaattcgaga aaaccgatat gggctggatt atctatccgg aaggtctgta tgatctgctg    1020

atgctgctgg atcgtgatta tggcaaaccg aatattgtga ttagcgaaaa tggtgcagcc    1080

ttcaaagatg aaattggtag caatggcaaa atcgaggata ccaaacgtat ccagtatctg    1140

aaagattatc tgacccaggc acatcgtgca attcaggatg gtgttaatct gaaagcatat    1200

tatctgtggt ccctgctgga taattttgaa tgggcatatg gttacaacaa acgctttggt    1260

attgtgcacg tgaattttga taccctggaa cgcaaaatca aagatagcgg ctattggtat    1320

aaagaggtga tcaaaaacaa cggctttggt acc                                 1353


<210> 6
<211> 451
<212> PRT
<213> Artificial Sequence

<220>
<223> Von Seq_5 abgeleitete Peptidsequnez der beta-Glucosidase BglA

<400> 6
Leu Glu Ser Lys Ile Thr Phe Pro Lys Asp Phe Ile Trp Gly Ser Ala
1               5                   10                  15
Thr Ala Ala Tyr Gln Ile Glu Gly Ala Tyr Asn Glu Asp Gly Lys Gly
            20                  25                  30
Glu Ser Ile Trp Asp Arg Phe Ser His Thr Pro Gly Asn Ile Ala Asp
        35                  40                  45
Gly His Thr Gly Asp Val Ala Cys Asp His Tyr His Arg Tyr Glu Glu
    50                  55                  60
Asp Ile Lys Ile Met Lys Glu Ile Gly Ile Lys Ser Tyr Arg Phe Ser
65                  70                  75                  80
Ile Ser Trp Pro Arg Ile Phe Pro Glu Gly Thr Gly Lys Leu Asn Gln

                85                  90                  95
Lys Gly Leu Asp Phe Tyr Lys Arg Leu Thr Asn Leu Leu Leu Glu Asn
            100                 105                 110
Gly Ile Met Pro Ala Ile Thr Leu Tyr His Trp Asp Leu Pro Gln Lys
        115                 120                 125
Leu Gln Asp Lys Gly Gly Trp Lys Asn Arg Asp Thr Thr Asp Tyr Phe
    130                 135                 140
Thr Glu Tyr Ser Glu Val Ile Phe Lys Asn Leu Gly Asp Ile Val Pro
145                 150                 155                 160
Ile Trp Phe Thr His Asn Glu Pro Gly Val Val Ser Leu Leu Gly His
                165                 170                 175
Phe Leu Gly Ile His Ala Pro Gly Ile Lys Asp Leu Arg Thr Ser Leu
            180                 185                 190
Glu Val Ser His Asn Leu Leu Leu Ser His Gly Lys Ala Val Lys Leu
        195                 200                 205
Phe Arg Glu Met Asn Ile Asp Ala Gln Ile Gly Ile Ala Leu Asn Leu
    210                 215                 220
Ser Tyr His Tyr Pro Ala Ser Glu Lys Ala Glu Asp Ile Glu Ala Ala
225                 230                 235                 240
Glu Leu Ser Phe Ser Leu Ala Gly Arg Trp Tyr Leu Asp Pro Val Leu
                245                 250                 255
Lys Gly Arg Tyr Pro Glu Asn Ala Leu Lys Leu Tyr Lys Lys Lys Gly
            260                 265                 270
Ile Glu Leu Ser Phe Pro Glu Asp Asp Leu Lys Leu Ile Ser Gln Pro
        275                 280                 285
Ile Asp Phe Ile Ala Phe Asn Asn Tyr Ser Ser Glu Phe Ile Lys Tyr
    290                 295                 300
Asp Pro Ser Ser Glu Ser Gly Phe Ser Pro Ala Asn Ser Ile Leu Glu
305                 310                 315                 320
Lys Phe Glu Lys Thr Asp Met Gly Trp Ile Ile Tyr Pro Glu Gly Leu
                325                 330                 335
Tyr Asp Leu Leu Met Leu Leu Asp Arg Asp Tyr Gly Lys Pro Asn Ile
            340                 345                 350
Val Ile Ser Glu Asn Gly Ala Ala Phe Lys Asp Glu Ile Gly Ser Asn
        355                 360                 365
Gly Lys Ile Glu Asp Thr Lys Arg Ile Gln Tyr Leu Lys Asp Tyr Leu
    370                 375                 380
Thr Gln Ala His Arg Ala Ile Gln Asp Gly Val Asn Leu Lys Ala Tyr
385                 390                 395                 400
Tyr Leu Trp Ser Leu Leu Asp Asn Phe Glu Trp Ala Tyr Gly Tyr Asn
                405                 410                 415
Lys Arg Phe Gly Ile Val His Val Asn Phe Asp Thr Leu Glu Arg Lys
            420                 425                 430
Ile Lys Asp Ser Gly Tyr Trp Tyr Lys Glu Val Ile Lys Asn Asn Gly
        435                 440                 445
Phe Gly Thr
    450